# FASCICULE DE BREVET EUROPEEN

(11) **EP 4 337 188 B1**
(45) Date de publication et mention de la délivrance du brevet: **09.04.2025**
(21) Numéro de dépôt: 22728244.9
(22) Date de dépôt: 10.05.2022
(51) Int. Cl.: A61K 31/20, A23L 33/12, A61K 47/64, A61K 47/69, A61P 1/00, A61P 43/00, A61K 31/201, A61K 31/202, A61K 47/50, A61K 31/19, A61K 31/198, A61K 31/385, A61K 31/575, A61K 31/375, A61K 31/185, A61K 31/197

(54) **COMPOSITIONS ET LEUR UTILISATION POUR RETABLIR LA PERMEABILITE INTESTINALE ET/OU PREVENIR OU LUTTER CONTRE DES MALADIES MULTIFACTORIELLES**
ZUSAMMENSETZUNGEN UND IHRE VERWENDUNG ZUR WIEDERHERSTELLUNG DER DARMDURCHLÄSSIGKEIT UND/ODER ZUR VORBEUGUNG ODER BEKÄMPFUNG VON MULTIFAKTORIELLEN KRANKHEITEN
COMPOSITIONS AND THEIR USE IN RESTORING INTESTINAL PERMEABILITY AND/OR PREVENTING OR COMBATING MULTIFACTORIAL DISEASES

(30) Priorité: 10.05.2021 FR 2104928
(43) Date de publication de la demande: 20.03.2024
(73) Titulaire: PLL THERAPEUTICS, 33270 Bouliac (FR)
(72) Inventeur: ZAMBAUX, Jean-Pascal, 33270 BOULIAC (FR)
(74) Mandataire: Aquinov
(86) Numéro de dépôt international: PCT/EP2022/062638
(87) Numéro de publication internationale: WO 2022/238403

(56) Documents cités:
- EP-B1- 0 792 167
- WO-A1-2016/097618
- FR-A1- 2 886 153
- FR-A1- 2 905 868
- FR-A1- 3 135 393
- JP-B2- 4 128 618
- US-B2- 7 427 600

## Description

### Domaine technique

L'invention concerne la prévention et/ou le traitement de maladies multifactorielles. En particulier l'invention est relative à des compositions comprenant plusieurs molécules spécifiques sélectionnées et leur utilisation pour diminuer la perméabilité intestinale et/ou rétablir la fonction de la perméabilité intestinale normale et/ou pour prévenir ou lutter contre différentes maladies multifactorielles impliquant une perméabilité intestinale.

### Etat de l'art

La plupart des maladies et en particulier des maladies chroniques sont multifactorielles. Elles font toujours intervenir une composante génétique et des composantes immunologiques et toxiques liées à « l'environnement », c'est-à-dire à des bactéries, des virus, des agents chimiques, etc.

Parmi les maladies chroniques multifactorielles connues, on peut citer en particulier les maladies dégénératives comme par exemple la maladie de Parkinson, la maladie d'Alzheimer, les effets post AVC inflammatoires, la maladie de Charcot (ou sclérose latérale amyotrophique), la maladie de Huntington, etc.

Les maladies dégénératives sont des pathologies qui entraînent une dégradation progressive d'un ou plusieurs organes. Souvent d'origine génétique, elles peuvent être déclenchées après une forte et longue exposition à des produits biologiques et/ou toxiques. En effet, tout passage excessif de constituants bactériens et/ou autres toxines à travers les muqueuses, entraîne une activation immunitaire, qui, chez les malades atteints d'affections chroniques, reprend toujours la voie de stimulation de clones auto réactifs s'attaquant aux tissus cibles (dégénérescence). Les maladies dégénératives sont donc intimement liées à une hyperperméabilité intestinale, comme beaucoup de maladies infectieuses et notamment de maladies virales.

Dans un intestin humain sain, de petites particules (< 4 Angstrom de rayon) peuvent migrer à travers les pores des Claudines qui sont le composant le plus important des jonctions serrées, mais des particules allant jusqu'à 10-15 Angstrom (3,5LDa) peuvent transiter par la voie d'absorption de l'espace paracellulaire. L'intestin présente normalement une certaine perméabilité, qui permet aux nutriments de traverser l'intestin, tout en maintenant une fonction barrière pour empêcher les substances potentiellement nocives de quitter l'intestin et de migrer plus largement vers le corps.

La flore intestinale interagit en permanence avec le système immunitaire intestinal, avec lequel elle est en contact. L'interaction entre la masse considérable d'antigènes constituée par la microflore intestinale et le système immunitaire intestinal est extrêmement complexe, mais dans un organisme en équilibre, cela entretient un « bruit de fond immunitaire », équilibre indispensable entre les bactéries de la flore et le système immunitaire qui les régule.

Une flore déséquilibrée, que l'on appelle une dysbiose, entraine une réponse exacerbée du système immunitaire intestinal, augmentant l'inflammation, ce qui a pour conséquence une altération des jonctions serrées. Le passage de fragments de parois de bactéries détruites par l'intestin, et passant dans la circulation générale, les endotoxines, s'accompagne de la sécrétion importante par l'organisme de cytokines inflammatoires, et si l'arrivée d'endotoxines se fait de manière massive, peuvent être le point de départ de choc septique, de détresse respiratoire, d'ischémie rénale ou d'orage cytokinique. Le dosage dans le sang des endotoxines est d'ailleurs un marqueur fiable d'une hyperperméabilité intestinale.

Les causes d'une hyperperméabilité intestinale sont extrêmement nombreuses. Toute perturbation du biotope intestinal, toute dysbiose, entraine une réponse du système immunitaire, avec libération de cytokines inflammatoires, qui a pour conséquence un état inflammatoire de la muqueuse de l'intestin grêle et du colon, avec altération de l'épithélium. Les causes de cette dysbiose peuvent être notamment :
- Le stress, qui réduit fortement le volume et la qualité de tous les sucs digestifs,
- Des chocs affectifs et psychiques,
- Des efforts importants ou des chocs physiques
- Les antibiothérapies,
- Les infections virales, bactériennes ou par des parasites,
- Des radiations
- Des agents chimiques toxiques : pollution de l'air, de l'eau, des aliments ; pesticides et métaux lourds ; des médicaments (statines, anti-inflammatoires, immunodépresseurs, etc.).

L'hyperperméabilité intestinale, en plus de participer au déclenchement de maladies dégénératives chez des patients prédisposés génétiquement, est à l'origine de maladies inflammatoires, infectieuses et auto-immunes, mais également d'allergies et d'intolérances alimentaires très invalidantes.

Actuellement, les solutions proposées pour prévenir ou lutter contre une dysbiose consistent essentiellement à administrer des probiotiques et/ou des prébiotiques, mais leur efficacité est très variable et dépend de la flore intestinale de base du patient.

Par ailleurs, en ce qui concerne spécifiquement les maladies dégénératives, celles-ci sont souvent très lourdes pour le patient. Certains symptômes peuvent être réduits, mais ces maladies ne sont jamais totalement guéries et il n'existe actuellement aucun traitement satisfaisant. De nombreux agents chimiques ont des propriétés thérapeutiques mais soit leur toxicité, soit leur durée de vie, soit leur élimination rapide les rendent impropre pour le traitement de maladies dégénératives.

En effet, alors qu'elles sont multifactorielles, les traitements actuels ne visent qu'un seul des facteurs à l'origine de ces maladies, et ne cherchent pas à agir sur l'hyperperméabilité intestinale. Par exemple, la maladie de Charcot est liée à de nombreux facteurs, en particulier à un stress oxydatif, un dysfonctionnement mitochondrial, une neuro-inflammation, une excitotoxicité, une dysfonction et une dégénérescence oligodendrocytaire, une protéostase altérée, un système de réparation de l'ADN altéré, des altérations dans l'ARN nucléocytoplasmique et dans l'ARN lié aux protéines de transport, transport axonal défectueux, transport vésiculaire défectueux. Or, actuellement, le traitement préconisé pour la maladie de Charcot est le Riluzole^{®} qui agit uniquement sur l'inhibition de la libération du glutamate pour lutter contre l'excitotoxicité, et en outre aucune action sur la perméabilité intestinale n'est envisagée. Le constat est identique pour les autres maladies dégénératives.

Il existe donc un besoin pour des compositions capables d'agir sur différents facteurs pour prévenir et/ou lutter contre les effets d'une hyperperméabilité intestinale et les maladies multifactorielles associées, en particulier contre les maladies dégénératives, auto-immunes, inflammatoires ou infectieuses.

### Résumé de l'invention

Pour répondre à ce besoin, l'invention propose une composition qui à la fois :
- réduit le processus inflammatoire
- diminue la perméabilité intestinale
- présente un effet anti-oxydant
- présente un effet anti-radicalaire
- présente un effet antibactérien, antiviral et antifongique,
- et préférentiellement rééquilibre les métabolismes neurologiques provoquant la dégénérescence des neurones et moto neurones.

A cet effet, l'invention a pour objet une composition comprenant au moins les molécules suivantes :
- acide oléique,
- acide palmitique,
- acide laurique,
- acide linoléique,
- acide azélaïque,
- farnésyl cystéine,
- acide palmitoléïque,
- cholestérol,
- acide thioctique,
- acide myristique,
- acide orotique,
- acide acétique,
- acide butyrique,
- acide lactique,
- acide propionique,
et/ou un sel et/ou un ester et/ou un anhydride d'une ou plusieurs de ces molécules.

Avantageusement, ces molécules agissent chacune sur des facteurs différents permettant de façon combinée d'agir sur le processus inflammatoire, la perméabilité intestinale, les agents chimiques toxiques, les radicaux libres et les microorganismes toxiques. Les molécules agissent ensemble pour permettre de rétablir la fonction de la perméabilité intestinale perturbée par un déséquilibre du microbiote.

Pour augmenter l'efficacité et la biodisponibilité de ces molécules, certaines sont préférentiellement conjuguées à des polymères spécifiques et d'autres, non conjugables directement à ces polymères, sont préférentiellement sous forme de co-polymères ou encapsulées dans des micelles. L'invention a également pour objet un procédé de fabrication de ces compositions.

Les compositions selon l'invention peuvent être utilisées comme médicament seules ou combinées à l'utilisation d'au moins une autre composition, et l'invention vise aussi les compositions pour leur utilisation comme médicament.

D'autres caractéristiques et avantages ressortiront de la description détaillée de l'invention, des exemples et des figures qui vont suivre.

### Brève description des Figures

[Fig. 1a] représente un schéma de polymérisation : Le L-Met NCA et le L-Lys NCA (trifluoroacétate) sont mélangés et polymérisés pour obtenir le copolymère (Poly-(L-Lysine-co-methionine). (DMF = dimethylformamide).
[Fig. 1b] représente un schéma de déprotection : le copolymère de la figure 1a est déprotégé pour obtenir un Poly-(L-Lysine-co-methionine) sous forme de sel de bromure.
[Fig. 2a] représente un schéma de polymérisation Le L-Cys NCA (trityle) et le L-Lys NCA (trifluoroacétate) sont mélangés et polymérisés pour obtenir le copolymère Poly-(L-lysine-co-L-cystéine).

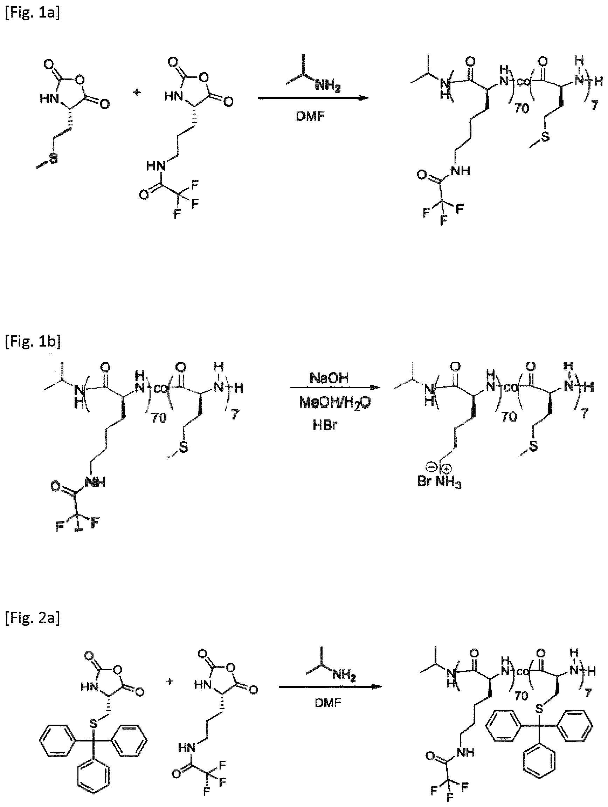

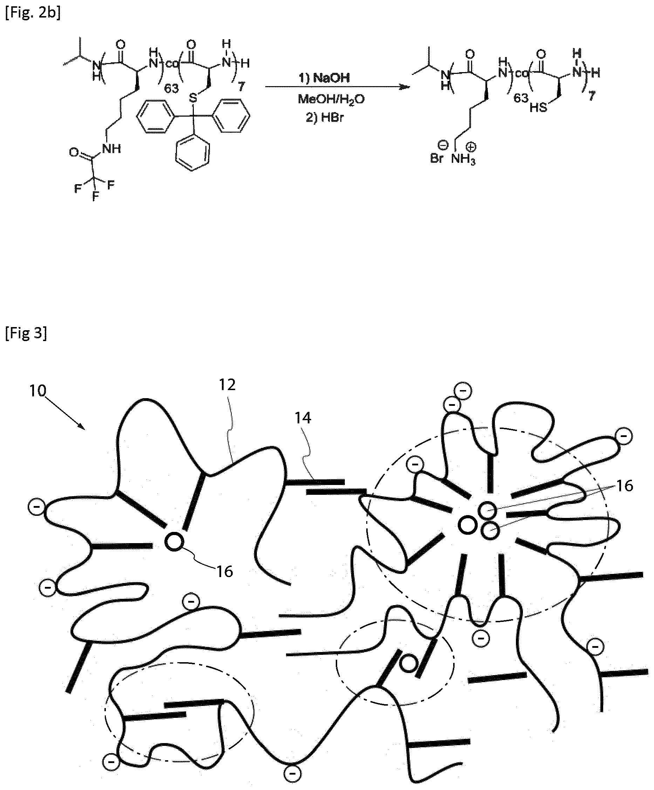

[Fig2 b] représente un schéma de déprotection : le copolymère de la figure 2a est déprotégé pour obtenir un Poly-(L-lysine-co-L-cystéine) sous forme de sel de bromure. (DMF = dimethylformamide).
[Fig3] représente un schéma de formations de micelles selon un mode de réalisation de l'invention : 10 = micelles ; 12= polymère (par exemple) poly-lysine ; 14 = molécules conjuguées de façon covalente (par exemple acides gras) ; 16 = molécules hautement hydrophobes non conjugables à la polylysine (par exemple cholestérol, farnésyl-cystéine, co-enzyme Q10).

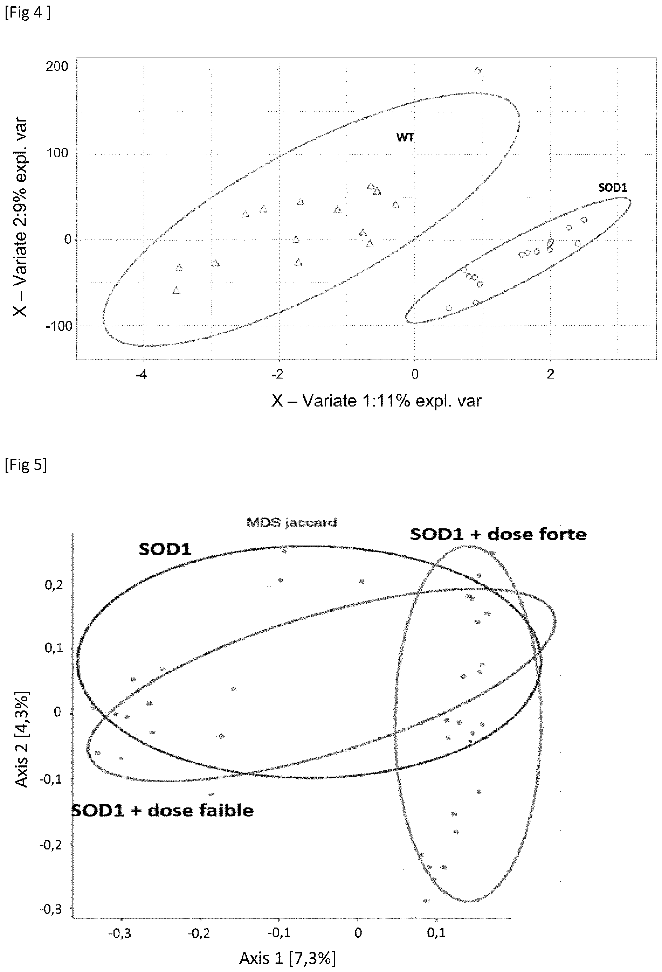

[Fig.4] est une représentation graphique d'une analyse PLS-DA de la comparaison du microbiote des souris WT et SOD1
[Fig 5] est une représentation graphique d'une analyse comparative de la béta diversité du microbiote intestinal de souris via la méthode MDS - Jaccard en fonction du traitement.

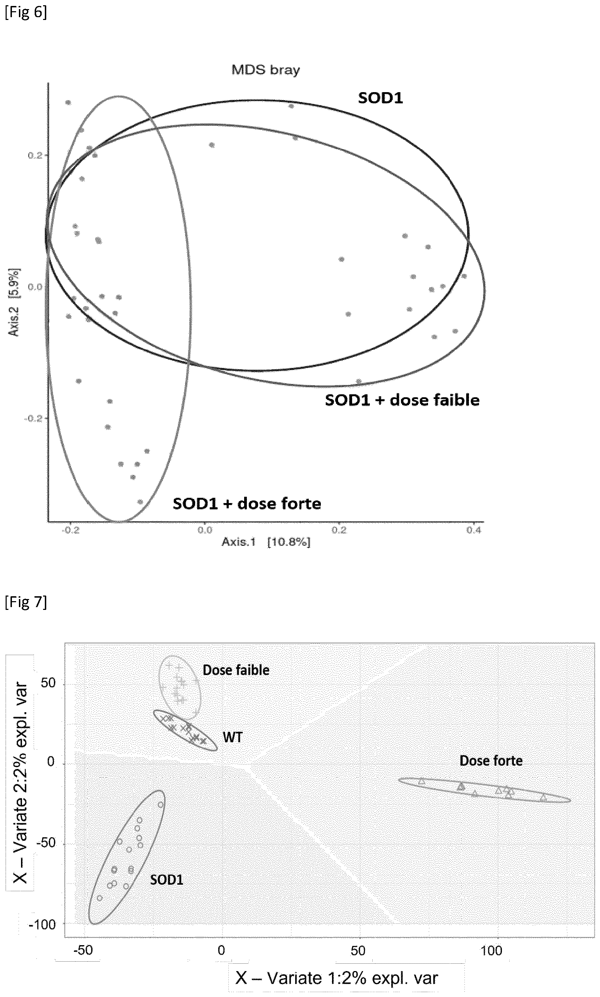

[Fig 6] est une représentation graphique d'une analyse comparative de la béta diversité du microbiote intestinal de souris via la méthode MDS - Bray Curtis en fonction du traitement.
[Fig 7] est une représentation graphique d'une analyse comparative du microbiote intestinal de souris en fonction du traitement via une analyse PLS-DA.

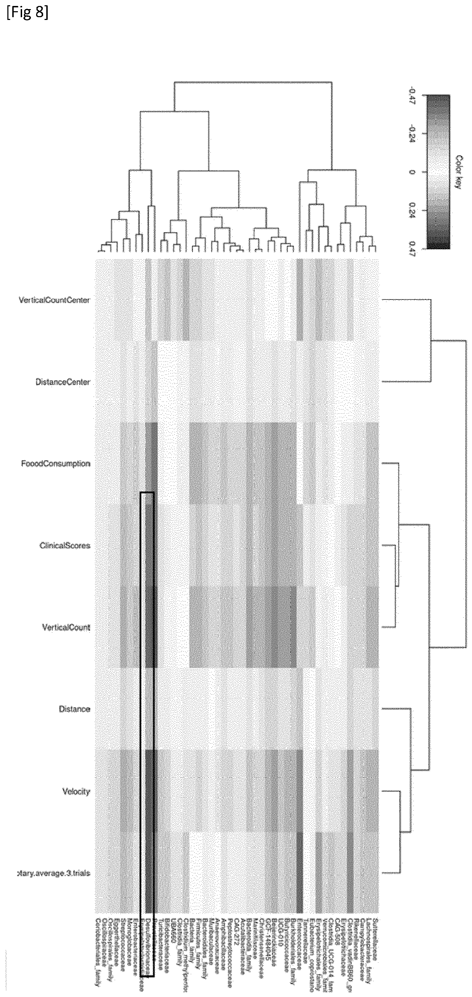

[Fig 8] est une représentation graphique d'une analyse de corrélation entre les souris WT ou SOD1 en fonction des familles bactériennes.

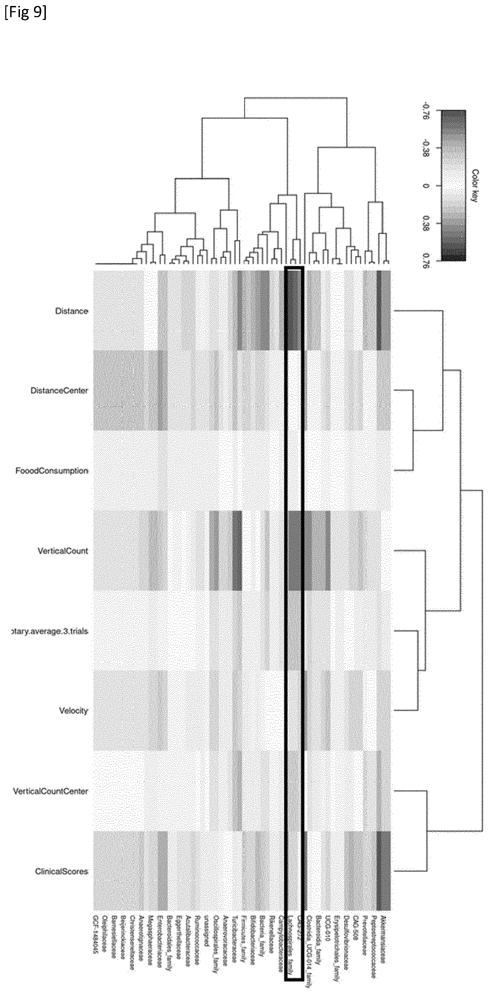

[Fig 9] est une représentation graphique d'une analyse de corrélation entre les souris SOD1 traitées ou non traitées à faible dose.

### Description détaillée de l'invention

### Définition

Par « animal » au sens de l'invention, on entend tout animal à l'exception de l'être humain. Il peut s'agir en particulier de mammifères.

Par « conjugué amphiphile » au sens de l'invention on entend un conjugué formé par une molécule X hydrophobe et un polymère Y hydrophile, le conjugué présentant ainsi une caractéristique amphiphile.

Par « molécule X conjuguée à un polymère Y» au sens de l'invention, on entend une molécule X lié par une liaison covalente à un polymère Y, ladite liaison pouvant être préférentiellement une liaison amide, urée ou carbamate en fonction de la nature chimique de la molécule X et du polymère Y.

### Composition C1

La présente invention a donc pour objet une composition C1 comprenant au moins les molécules actives suivantes :
- acide oléique,
- acide palmitique,
- acide laurique,
- acide linoléique,
- acide azélaïque,
- farnésyl cystéine,
- acide palmitoléïque,
- cholestérol,
- acide thioctique,
- acide myristique,
- acide orotique,
- acide acétique,
- acide butyrique,
- acide lactique,
- acide propionique,
et/ou un sel et/ou un ester et/ou un anhydride d'une ou plusieurs de ces molécules.

La composition C1 peut éventuellement comprendre également l'acide pyruvique et/ou un sel et/ou un ester et/ou un anhydride.

Pour toutes les molécules actives présentes dans la composition C1, lorsqu'elles sont mentionnées dans la présente demande, il peut s'agir des molécules (exemple acide butyrique) en tant que telles et/ou d'un sel (exemple : butyrate) et/ou d'un ester et/ou d'un anhydride de ces molécules (exemple : ester d'acide butyrique) et/ou un anhydride.

### Préférentiellement :

- la quantité d'acide butyrique dans la composition C1 est supérieure à celle de chacune des autres molécules prises individuellement, et/ou
- la quantité d'acide thioctique dans la composition C1 est supérieure à celle de chacune des autres molécules prises individuellement, sauf de l'acide butyrique, et/ou
- la quantité d'acide laurique dans la composition C1 est supérieure à celle de de chacune des molécules suivantes prises individuellement :, l'acide palmitique, l'acide linoléique, l'acide azélaïque, farnésyl cystéine, l'acide palmitoléïque, le cholestérol, l'acide myristique, l'acide orotique,
- la quantité d'acide oléique d'une part et d'acide lactique d'autre part dans la composition C1 est supérieure à celle de chacune des molécules suivantes prises individuellement : l'acide palmitique, l'acide laurique, l'acide linoléique, l'acide azélaïque, farnésyl cystéine, l'acide palmitoléïque, le cholestérol, l'acide myristique, l'acide orotique, l'acide acétique.

Préférentiellement, chacune des molécules actives dans la composition C1 représente entre 0,5 E-05 M et 10 E-05 M.

Selon un mode de réalisation, pour améliorer la solubilité, l'efficacité et la biodisponibilité des molécules de la composition C1, au moins une des molécules de la composition choisies parmi l'acide oléique, l'acide palmitique, l'acide laurique, l'acide linoléique, l'acide azélaïque, l'acide palmitoléïque, l'acide thioctique, l'acide myristique, l'acide orotique, l'acide acétique, l'acide butyrique, l'acide lactique, l'acide propionique, les sels de ces acides, les esters de ces acides et les anhydrides de ces acides, est conjuguée de façon covalente à au moins une molécule d'un polymère choisi parmi la poly-lysine, le poly-éthylène-glycol, la poly-ornithine, la poly-arginine et la poly-histidine.

En effet, les molécules choisies parmi l'acide oléique, l'acide palmitique, l'acide laurique, l'acide linoléique, l'acide azélaïque, l'acide palmitoléïque, l'acide thioctique, l'acide myristique, l'acide orotique, l'acide acétique, l'acide butyrique, l'acide lactique, l'acide propionique, les sels de ces acides, les esters de ces acides et les anhydrides de ces acides, sont toutes conjugables de façon covalente avec un polymère choisi parmi la poly-lysine, le poly-éthylène-glycol, la poly-ornithine, la poly-arginine et la poly-histidine, notamment par une liaison amide, urée ou carbamate.

Selon une variante, particulièrement adaptée toutes les molécules choisies parmi l'acide oléique, l'acide palmitique, l'acide laurique, l'acide linoléique, l'acide azélaïque, l'acide palmitoléïque, l'acide thioctique, l'acide myristique, l'acide orotique, l'acide acétique,l'acide butyrique, l'acide lactique, l'acide propionique, les sels de ces acides et les esters de ces acides, présentes dans la composition C1 selon l'invention sont conjuguées de façon covalente à au moins une molécule d'un polymère choisi parmi la poly-lysine, le poly-éthylène-glycol, la poly-ornithine, la poly-arginine et la poly-histidine.

Parmi l'ensemble des molécules à conjuguer, 2 d'entre elles sont hautement hydrophobes (valeurs LogP supérieures à 1, préférentiellement supérieures à 2 et préférentiellement supérieures à 3) et chimiquement non appropriées pour une conjugaison covalente, soit en raison de l'absence de groupes fonctionnels réactifs, soit en raison d'incompatibilités chimiques. Il s'agit du cholestérol et du farnesyl-cystéine. D'autre part, considérant à la fois le caractère hydrophile de certains polymères (comme la poly-lysine, le poly-éthylène-glycol, la poly-ornithine, la poly-arginine ou la poly-histidine) et le caractère hydrophobe de la plupart des molécules convenant à la conjugaison covalente (l'acide oléique, l'acide palmitique, l'acide laurique, l'acide linoléique, l'acide azélaïque, l'acide palmitoléïque, l'acide thioctique, l'acide myristique, l'acide orotique, l'acide acétique,l'acide butyrique, l'acide lactique, l'acide propionique), selon l'invention ces conjugués particuliers se comportent comme des surfactants en solution aqueuse. De cette manière, certains de ces conjugués (amphiphiles) agissent comme des micelles polymères en milieu aqueux, générant une poche hydrophobe, capable d'encapsuler des espèces hydrophobes, améliorant ainsi considérablement leur solubilité et leur stabilité en solution aqueuse.

Le farnésyl cystéine et le cholestérol, et les esters, les sels et les anhydrides de farnésyl cystéine ou de cholestérol ne peuvent pas se lier de façon covalente, ne peuvent pas être conjugués, à un polymère choisi parmi la poly-lysine, le poly-éthylène-glycol, la poly-ornithine, la poly-arginine et la poly-histidine. Aussi, préférentiellement, le farnésyl cystéine et/ou le cholestérol, et/ou leurs esters, présents dans la composition C1 sont encapsulés dans des micelles. Ainsi, la composition C1 comprend préférentiellement des micelles dans lesquelles sont encapsulées au moins le farnésyl cystéine et/ou le cholestérol et/ou un de leurs esters et/ou sels et/ou anhydrides. Selon un mode de réalisation particulièrement adapté, au moins une des micelles est formée par des conjugués amphiphiles constitués chacun par au moins une molécule hydrophobe conjuguée de façon covalente à une molécule d'un polymère choisi parmi la poly-lysine, le poly-éthylène-glycol, la poly-ornithine, la poly-arginine et la poly-histidine. Encore plus préférentiellement, tel que représenté sur la figure 3, au moins une des micelles 10 est formée par des conjugués amphiphiles constitués chacun par au moins une molécule hydrophobe 14 préférentiellement choisie parmi les acides gras de la composition et notamment au moins une molécule choisie parmi l'acide oléique, l'acide palmitique, l'acide laurique, l'acide linoléique, l'acide palmitoléïque, l'acide myristique, les sels de ces acides, les esters de ces acides et les anhydrides de ces acides, conjuguée de façon covalente à une molécule d'un polymère 12 choisi parmi la poly-lysine, le poly-éthylène-glycol, la Poly-ornithine, la poly-arginine et la poly-histidine, lesdits micelles encapsulant le farnésyl cystéine et/ou le cholestérol et/ou un de leurs esters et/ou sels et/ou anhydrides 16. En effet, l'acide oléique, l'acide palmitique, l'acide laurique, l'acide linoléique, l'acide azélaïque, l'acide palmitoléïque, l'acide myristique, l'acide propionique, les sels de ces acides, les esters de ces acides et les anhydrides de ces acides, sont hydrophobes et la poly-lysine, le poly-éthylène-glycol, la poly-ornithine, la poly-arginine et la poly-histidine sont des polymères hydrophiles.

Ainsi, la composition C1 comprend préférentiellement :
- des conjugués de l'acide oléique, l'acide palmitique, l'acide laurique, l'acide linoléique, l'acide azélaïque, l'acide palmitoléïque, l'acide thioctique, l'acide myristique, l'acide orotique, l'acide acétique, l'acide butyrique, l'acide lactique, l'acide propionique, les sels de ces acides, les esters de ces acides et les anhydrides de ces acides, avec la poly-lysine, le poly-éthylène-glycol, la poly-ornithine, la poly-arginine ou la poly-histidine, et
- des micelles formées par au moins un de ces conjugués, lesdits micelles encapsulant le farnésyl cystéine et le cholestérol.

Cette configuration permet en particulier :
- d'augmenter la demi-vie des molécules actives de la composition dans l'organisme,
- de cibler les tissus ou les cellules sur lesquelles les molécules de composition doivent agir,
- de permettre aux molécules actives de la composition de passer la barrière hémato encéphalique et de pénétrer dans les cellules, notamment dans les neurones et les motoneurones,
- d'augmenter la stabilité et la biodisponibilité de l'actif.

L'efficacité de la composition est donc plus importante et il est possible d'administrer des doses plus faibles et de réduire la toxicité aigüe ou chronique des molécules actives contenues dans la composition.

Préférentiellement, le ou les polymère(s) conjugués aux molécules sont choisis parmi la poly-L-lysine, le poly-éthylène-glycol, la poly-L-ornithine, la poly-L-arginine et la poly-L-histidine.

Selon un mode de réalisation il s'agit de la poly-lysine, préférentiellement de la poly-L-lysine. La polylysine est préférentiellement linéaire. En particulier la poly-lysine utilisée est une epsilon poly-L-lysine de poids moléculaire compris entre 12 000 et 20 000 Da.

Ainsi, selon une variante, la composition C1 comprend au moins :
- A. les conjugués suivants, chaque conjugué étant constitué par une molécule liée de façon covalente à une poly-lysine :
- un ou plusieurs conjugués Oléyl-Poly-L-Lysine
- un ou plusieurs conjugués Palmitic-Poly-L-Lysine
- un ou plusieurs conjugués Lauryl-Poly-L-Lysine
- un ou plusieurs conjugués Azélayl-Poly-L-Lysine
- un ou plusieurs conjugués Palmitoléyl-Poly-L-Lysine
- un ou plusieurs conjugués Thioctyl-Poly-L-Lysine
- un ou plusieurs conjugués Myristyl-Poly-L-Lysine
- un ou plusieurs conjugués Orotyl-Poly-L-Lysine
- un ou plusieurs conjugués Acétate-Poly-L-Lysine
- un ou plusieurs conjugués Butyrate-Poly-L-Lysine
- un ou plusieurs conjugués Lactate-Poly-L-Lysine
- un ou plusieurs conjugués Propionate-Poly-L-Lysine
- un ou plusieurs conjugués Linoléyl-Poly-L-Lysine, et
- B. du farnésyl cystéine et du cholestérol, et/ou un ester de ces molécules, encapsulées dans des micelles, préférentiellement dans des micelles formées par un ou plusieurs des conjugués listés dans la liste A.

Dans cette composition, la poly-L-Lysine peut être remplacée par une autre poly-lysine ou par le poly-éthylène-glycol, une poly-L-ornithine, une poly-L-arginine ou une poly-L-histidine.

La composition C1 selon l'invention peut se présenter sous forme liquide ou sous forme solide. Lorsqu'elle est sous forme liquide la composition comprend au moins de l'eau et les constituants ciavant mentionnés. La forme solide est préférentiellement obtenue à partir de la forme liquide, de façon préférée par lyophilisation. Ainsi, lorsque la composition C1 sous forme liquide comprend des micelles et qu'elle est lyophilisée sous forme solide, les micelles se reforment lorsque la composition C1 sous forme solide est de nouveau mise dans une solution aqueuse.

La composition C1 comprend également préférentiellement des excipients pharmaceutiquement acceptables. Ces excipients peuvent être choisis en particulier pour répondre aux pH et osmolarité de solution injectables chez l'homme ou l'animal. Il peut s'agir par exemple d'acides ou de bases pour ajuster le pH ou de NaCl pour ajuster l'osmolarité.

La composition C1 selon l'invention est destinée à être administrée à l'être humain, ou à un animal et se présente par conséquent sous une forme adaptée à une telle administration. Lorsqu'elle se présente sous forme liquide, elle est préférentiellement adaptée à une administration par voie souscutanée ou par voie intraveineuse, notamment par voie intraveineuse en perfusion, et elle est conditionnée dans des contenants adaptés et connus de l'homme du métier pour conditionner ce type de produits. La composition C1 peut être aussi administrée sous forme liquide via une pompe, du type des pompes à insuline.

Lorsqu'elle se présente sous forme solide, elle est préférentiellement adaptée à une administration :
- par voie transcutanée et elle est préférentiellement formulée et conditionnée sous forme d'un patch, ou
- par voie nasale sous forme de poudre, ou
- par voie sublinguale sous forme de poudre ou de comprimé, ou
- par voie transmucosale et elle est préférentiellement formulée et conditionnée sous forme d'un comprimé muco-adhésif.

### Procédé de fabrication de la composition C1

La composition C1 selon l'invention peut être fabriquée par tout procédé adapté.

Si les molécules qui constituent la composition C1 sont utilisées telles quelles dans un solvant, elles peuvent être mélangées toutes ensemble dans ledit solvant.

Si les molécules dans la composition C1 sont conjuguées à des polymères pour une partie et dans des micelles pour d'autres, le procédé de fabrication peut comprendre les étapes suivantes :
- a. créer un premix (pré-mélange) amphiphile : mélanger dans une solution aqueuse un ou plusieurs conjugués choisis parmi :
- un ou plusieurs conjugués Oléyl-Poly-L-Lysine
- un ou plusieurs conjugués Palmitic-Poly-L-Lysine
- un ou plusieurs conjugués Lauryl-Poly-L-Lysine
- un ou plusieurs conjugués Azélayl-Poly-L-Lysine
- un ou plusieurs conjugués Palmitoléyl-Poly-L-Lysine
- un ou plusieurs conjugués Thioctyl-Poly-L-Lysine
- un ou plusieurs conjugués Myristyl-Poly-L-Lysine
- un ou plusieurs conjugués Orotyl-Poly-L-Lysine
- un ou plusieurs conjugués Acétate-Poly-L-Lysine
- un ou plusieurs conjugués Butyrate-Poly-L-Lysine
- un ou plusieurs conjugués Lactate-Poly-L-Lysine
- un ou plusieurs conjugués Propionate-Poly-L-Lysine,
- un ou plusieurs conjugués Linoléyl-Poly-L-Lysine, et
- b. ajouter dans le premix amphiphile au moins du farnésylcystéine et du cholestérol, et mettre sous agitation de façon à former des micelles formées par un ou plusieurs des conjugués du premix amphiphile encapsulant le farnésylcystéine et le cholestérol.

En effet, une variante de l'invention consiste à tirer parti de la nature amphiphile de la plupart des composants individuels, pour créer un prémélange amphiphile qui permet la solubilisation contrôlée des espèces hydrophobes. Le processus de solubilisation selon un mode de réalisation préféré consiste en une addition contrôlée des molécules hydrophobes aux prémélanges amphiphiles et à laisser le temps nécessaire à leur dissolution sous agitation.

La Poly-L-lysine peut être remplacée par une autre poly-lysine ou par le poly-éthylène-glycol, une poly-L-ornithine, une poly-L-arginine ou une poly-L-histidine.

Préférentiellement, l'agitation est réalisée pendant au moins 60 minutes, encore plus préférentiellement entre 5 et 20 minutes, et de façon préférée à une vitesse d'agitation inférieure ou égale à 900 tours par minute, en particulier à une vitesse d'agitation comprise entre 50 et 800 tours par minute.

Selon un mode de réalisation préféré, le procédé de fabrication selon l'invention comprend également une étape c. de séparation des phases soluble et insoluble, pour récupérer la phase soluble. Dans ce cas la phase insoluble et éliminée et la phase soluble constitue la composition C1 selon l'invention. En effet, un processus de séparation physique (filtration, ultrafiltration) est préférentiellement réalisé pour assurer l'isolement de la fraction soluble, contenant à la fois le prémélange amphiphile et les molécules solubilisées.

La composition C1 sous forme liquide peut ensuite être lyophilisée ou déshydratée pour être sous forme solide, préférentiellement par lyophilisation lente par exemple entre 12 et 36 heures.

Par ailleurs, si des conjugués molécule active-polymère de la composition C1 ne sont pas intégrés dans le premix de l'étape a, ceux-ci peuvent être ajoutés dans le mélange après l'étape b. c'est-à-dire après la formation des micelles et l'encapsulation du farnésylcystéine et du cholestérol.

Les conjugués molécule active-polymère peuvent être fabriqués par tout moyen connu de l'homme du métier pour conjuguer une molécule de façon covalente à un polymère en fonction de leur nature chimique. Ainsi l'acide laurique, l'acide myristique, l'acide palmitique, l'acide oléique, l'acide linoléique, l'acide orotique, l'acide azélaïque, l'acide thioctique, l'acide acétique, l'acide palmitoléique, l'acide butyrique, l'acide lactique, l'acide propionique, et l'acide oléique sont conjugués au polymère (préférentiellement poly-L-Lysine) par une liaison amide.

Des exemples de réalisation de liaisons amides, urée et carbamates sont décrits par exemple dans :
- Ryser HJ, Shen WC. Conjugation of methotrexate to poly (L-lysine) as a potential way to overcome drug resistance. Cancer. 1980 Mar 15;45(5 Suppl):1207-11 (liaisons amide),
- Zhuxian Z, Jianbin T, Qihang S, William J. A multifunctional PEG-PLL drug conjugate forming redox-responsive nanoparticles for intracellular drug delivery Issue 38, 2015. Journal of Materials Chemistry B (liaisons amide),
- Scheper V, Wolf M, Scholl M, Kadlecova Z, Perrier T, Klok HA, Saulnier P, Lenarz T, Stöver T. Potential novel drug carriers for inner ear treatment: hyperbranched polylysine and lipid nanocapsules. Nanomedicine (Lond). 2009 Aug;4(6):623-35 (liaisons urée),
- Stéphanie Gac-Breton, Jean Coudane, Mahfoud Boustta & Michel Vert (2004) Norfloxacin-Poly(l-Lysine Citramide Imide) Conjugates and Structure-dependence of the Drug Release, Journal of Drug Targeting, 12:5, 297-307, (liaisons carbamate),
- Elmore, W. M. (2013). Nanoparticles Stabilized with MPEG-Polylysine Carbamate: Synthesis and Characterization, (liaisons carbamate),
- Ning-Ping Huang, Janos Vörös, Susan M. De Paul, Marcus Textor, and Nicholas D. Spencer. Biotin-Derivatized Poly(l-lysine)-g-poly(ethylene glycol): A Novel Polymeric Interface for Bioaffinity Sensing. Langmuir 2002 18 (1), 220-230 (liaisons carbamate).

### Utilisation de la composition C1

La composition C1 est une formulation générique particulièrement adaptée pour prévenir et/ou traiter les maladies multifactorielles, notamment les maladies chroniques multifactorielles, et en particulier les maladies impliquant une perméabilité intestinale.

Elle est capable d'agir de façon combinée :
- comme immuno-modulateur et régulateur de l'auto-immunité, en particulier grâce à la présence d'acide oléique, de l'acide palmitique, de l'acide palmitoléique,
- comme anti-inflammatoire, en particulier grâce à la présence de l'acide palmitique, de l'acide azélaïque, du farnésyl cystéine, de l'acide palmitoléique, de l'acide myristique,
- comme antibactérien, en particulier grâce à la présence de l'acide laurique, de l'acide azélaïque,
- comme antifongique, en particulier grâce à la présence de l'acide laurique,
- comme anti-radicalaire, en particulier grâce à la présence du farnésyl cystéine,
- comme antagoniste des protéines isoprénylées, en particulier grâce à la présence du farnésyl cystéine,
- sur la stabilisation des membranes cellulaires, en particulier grâce à la présence de cholestérol,
- comme piégeur de radicaux libres et de métaux, en particulier grâce à la présence d'acide thioctique.
- comme anti-viral, en particulier grâce à la présence d'acide myristique,
- comme substrat synergique cellulaire, en particulier grâce à la présence de butyrate,
- pour favoriser le développement de la flore intestinale, en particulier grâce à la présence d'acide orotique,
- pour réguler les muqueuses et la flore intestinale, en particulier grâce à la présence d'acétate, de lactate, de propionate et de Butyrate

Les différentes molécules présentes agissent en synergie et permettent notamment de rétablir la fonction de la perméabilité intestinale.

Ainsi, la composition C1 peut être utilisée pour rétablir la fonction de la membrane intestinale et/ou pour maintenir la perméabilité intestinale et/ou prévenir ou lutter contre une hyperperméabilité de la muqueuse intestinale.

Selon une variante, l'invention a pour objet l'utilisation d'une composition C1 comme complément alimentaire chez une personne saine ou un animal sain, en particulier pour diminuer la perméabilité intestinale de la personne ou de l'animal à qui elle est destinée.

L'intestin joue donc un rôle clef d'initiateur, de régulateur sur la chronicité des maladies. Ainsi, la composition C1 peut notamment être administrée à l'Homme ou l'animal pour prévenir ou lutter contre des maladies dans lesquelles l'intestin joue un rôle important, notamment des maladies chroniques, et en particulier des maladies chroniques dégénératives, telles que des maladies neurodégénératives telles que par exemple la maladie de Charcot. La composition C1 peut aussi être utilisée comme activateur du système immunitaire pour prévenir ou lutter contre des maladies auto immunes et infectieuses.

La composition peut être utilisée en particulier pour diminuer la chronicité des maladies chroniques.

Préférentiellement, la composition C1 est utilisée en prévention ou en traitement dès les premiers symptômes d'une hyperperméabilité intestinale ou d'une maladie chronique, de façon à empêcher le développement de la chronicité de ladite maladie et ainsi éviter l'orage inflammatoire qui découle d'une hyperperméabilité intestinale prolongée.

De façon spécifique, la composition C1 est particulièrement utile pour au moins :
- réduire le processus inflammatoire, et/ou
- réduire le processus auto-immun, et/ou
- diminuer la perméabilité intestinale, et/ou
- un effet anti-oxydant, et/ou
- un effet anti-radicalaire, et/ou
- un effet antibactérien et/ou antiviral/ou et antifongique.

L'invention a donc également pour objet une composition C1 selon l'invention pour son utilisation comme médicament, notamment chez l'Homme ou l'animal c'est-à-dire pour un sujet Humain ou animal.

En particulier l'invention a pour objet une composition C1 pour son utilisation dans la prévention et/ou le traitement d'une pathologie multifactorielle, en particulier une pathologie multifactorielle chronique, notamment une pathologie choisie parmi les maladies inflammatoires, les maladies neurodégénératives, les maladies bactériennes, les maladies virales, les maladies auto-immunes, les allergies et intolérances alimentaires.

Selon un mode de réalisation particulier, l'invention a pour objet une composition C1 pour son utilisation dans la prévention et/ou le traitement d'une pathologie choisie parmi l'eczéma, le psoriasis, les maladies inflammatoires chroniques intestinales (notamment maladie de Crohn et Coeliaque), la maladie d'Alzheimer, la maladie de Parkinson, la sclérose en plaque, la maladie de Charcot.

La composition C1 peut être utilisée seule ou avec une ou plusieurs autres compositions.

Selon un mode de réalisation, la composition C1 est préférentiellement utilisée avec une composition C2.

### Composition C2

La présente invention a donc également pour objet une composition C2 comprenant au moins les molécules actives suivantes :
- taurine,
- cystéine,
- méthionine
- spermine
- acide rétinoïque,
- acide pantothénique,
- biotine,
- co-enzyme Q10,
- Glutathion,
- acide ascorbique,
- GABA,
- alpha-tocophérol,
et/ou un sel et/ou un ester et/ou un anhydride d'une ou plusieurs de ces molécules.

Pour toutes les molécules actives présentes dans la composition C2, lorsqu'elles sont mentionnées dans la présente demande, il peut s'agir des molécules (exemple acide ascorbique) en tant que telles et/ou d'un sel (exemple : ascorbate) et/ou d'un ester de ces molécules(exemple : ester d'acide ascorbique). et/ou d'un anhydride (exemple : anhydride d'acide ascorbique).

### Préférentiellement :

- la quantité d'acide rétinoïque dans la composition C2 est supérieure à celle de chacune des autres molécules prises individuellement, et/ou
- la quantité de taurine, la quantité de méthionine et la quantité de GABA sont équivalentes dans la composition C2 et sont chacune supérieures à celle de chacune des autres molécules prises individuellement, sauf de l'acide rétinoïque et du coenzyme Q10.

Préférentiellement, chacune des molécules actives dans la composition C2 représente entre 6 E-05 M et 18 E-05 M.

Selon un mode de réalisation, pour améliorer la solubilité, l'efficacité et la biodisponibilité des molécules de la composition C2, au moins une des molécules de la composition choisies parmi la taurine, la spermine, l'acide pantothénique, la biotine, le Glutathion, l'acide ascorbique, le GABA, et l'alpha-tocophérol, les sels de ces molécules, les esters de ces molécules et les anhydrides de ces molécules, est conjuguée de façon covalente à au moins une molécule d'un polymère choisi parmi la poly-lysine, le poly-éthylène-glycol, la poly-ornithine, la poly-arginine et la poly-histidine.

En effet, les molécules choisies parmi la taurine, la spermine, l'acide pantothénique, la biotine, l'acide rétinoïque, le Glutathion, l'acide ascorbique, le GABA, et l'alpha-tocophérol, les sels de ces molécules, les esters de ces molécules et les anhydrides de ces molécules, sont toutes conjugables de façon covalente avec un polymère choisi parmi la poly-lysine, le poly-éthylène-glycol, la poly-ornithine, la poly-arginine et la poly-histidine, notamment par une liaison amide, urée ou carbamate.

Selon une variante, particulièrement adaptée toutes les molécules choisies parmi la taurine, la spermine, l'acide rétinoïque, l'acide pantothénique, la biotine, le Glutathion, l'acide ascorbique, le GABA, et l'alpha-tocophérol, les sels de ces molécules, les esters de ces molécules et les anhydrides de ces molécules, présentes dans la composition C1 selon l'invention sont conjuguées de façon covalente à au moins une molécule d'un polymère choisi parmi la poly-lysine, le poly-éthylène-glycol, la poly-ornithine, la poly-arginine et la poly-histidine.

La cystéine et la méthionine, et les esters, les sels et les anhydrides de cystéine ou de méthionine ne peuvent pas se lier de façon covalente, ne peuvent pas être conjugués, à un polymère choisi parmi la poly-lysine, le poly-éthylène-glycol, la poly-ornithine, la poly-arginine et la poly-histidine. Aussi, préférentiellement, la cystéine et/ou la méthionine, et/ou leurs esters et/ou sels, présents dans la composition C2 sont liés audit polymère par copolymérisation pour former un copolymère. Préférentiellement, la cystéine et/ou la méthionine, et/ou leurs esters et/ou sels et/ou anhydrides sont inclus dans l'épine dorsale polypeptidique du polymère pour former un copolymère.

La copolymérisation peut être réalisée notamment par la mise en œuvre d'une étape de polymérisation (par exemple L-Met NCA ou L-Cys NCA et L-Lys NCA sont mélangés et polymérisés pour obtenir un copolymère) puis une étape de déprotection du copolymère obtenu après polymérisation. Des exemples sont présentés sur les figures 1a, 1b (Poly-(L-Lysine-co-methionine), 2a et 2b (Poly-(L-lysine-co-L-cystéine) :
- 1a : Polymérisation : Le L-Met NCA et le L-Lys NCA (trifluoroacétate) sont mélangés et polymérisés dans le rapport approprié pour obtenir le copolymère aléatoire approprié,
- 1b : Déprotection du PLys-TFA : Le copolymère précédent est déprotégé pour obtenir du PLys sous forme de sel de bromure.
- 2a : Polymérisation : Le L-Cys NCA (trityle) et le L-Lys NCA (trifluoroacétate) sont mélangés dans le rapport approprié pour obtenir le copolymère aléatoire approprié.
- 2b : Déprotection de Lys-TFA : Le copolymère précédent est déprotégé pour obtenir L-Lys sous forme de sel de bromure et L-Cys sous sa forme déprotégée.

Ainsi, dans la composition C2, la cystéine et la méthionine sont chacune conjuguées à une molécule d'un polymère choisi parmi la poly-lysine, le poly-éthylène-glycol, la Poly-ornithine, la poly-arginine et la poly-histidine, par polymérisation, ladite molécule et ledit polymère formant un copolymère. Préférentiellement, les copolymères ont entre 5 et 20% de cystéine ou de méthionine et entre 80 et 95% de polymère (choisi parmi la poly-lysine, le poly-éthylène-glycol, la Poly-ornithine, la poly-arginine et la poly-histidine). Selon une variante préférée, les copolymères ont 10 % de cystéine ou de méthionine et 90% de polymère (rapport de 1 pour 10).

Parmi l'ensemble des molécules à conjuguer, une d'entre elles est hautement hydrophobe (valeurs LogP supérieures à 1, préférentiellement supérieures à 2 et préférentiellement supérieures à 3) et chimiquement non appropriée pour une conjugaison covalente, en raison de l'absence de groupes fonctionnels réactifs et en raison d'incompatibilités chimiques. Il s'agit du co-enzyme Q10. D'autre part, considérant à la fois le caractère hydrophile de certains polymères (comme la poly-lysine, le poly-éthylène-glycol, la poly-ornithine, la poly-arginine ou la poly-histidine) et le caractère hydrophobe de certaines molécules convenant à la conjugaison covalente (alpha-tocophérol, acide rétinoïque), selon l'invention ces conjugués particuliers se comportent comme des surfactants en solution aqueuse. De cette manière, certains de ces conjugués (amphiphiles) agissent comme des micelles polymères en milieu aqueux, générant une poche hydrophobe, capable d'encapsuler le coenzyme Q10 hydrophobes, améliorant ainsi considérablement leur solubilité et leur stabilité en solution aqueuse.

Le co-enzyme Q10, et les esters, les sels et anhydrides de co-enzyme Q10 ne peuvent en effet pas se lier de façon covalente, ne peuvent pas être conjugués, à un polymère choisi parmi la poly-lysine, le poly-éthylène-glycol, la poly-ornithine, la poly-arginine et la poly-histidine. Aussi, préférentiellement le co-enzyme Q10 et/ou les esters et sels de co-enzyme Q10 sont encapsulés dans des micelles. Ainsi, la composition C2 comprend préférentiellement des micelles dans lesquelles sont encapsulées au moins le co-enzyme Q10 et/ou des esters et/ou des sels et/ou des anhydrides de co-enzyme Q10.

Selon un mode de réalisation particulièrement adapté, au moins une des micelles est formée par des conjugués amphiphiles constitués chacun par au moins une molécule hydrophobe conjuguée de façon covalente à une molécule d'un polymère choisi parmi la poly-lysine, le poly-éthylène-glycol, la poly-ornithine, la poly-arginine et la poly-histidine. Encore plus préférentiellement, tel que représenté sur la figure 3, au moins une des micelles 10 est formée par des conjugués amphiphiles constitués chacun par au moins une molécule 14 choisie parmi la taurine, la spermine, l'acide pantothénique, la biotine, le Glutathion, l'acide ascorbique, le GABA, et l'alpha-tocophérol, les sels de ces molécules, les esters de ces molécules et les anhydrides de ces molécules, conjuguée de façon covalente à une molécule d'un polymère 12 choisi parmi la poly-lysine, le poly-éthylène-glycol, la Poly-ornithine, la poly-arginine et la poly-histidine, encapsulant le co-enzyme Q10 et/ou des esters et/ou des sels et/ou des anhydrides du co-enzyme Q10 16. En effet, la taurine, la spermine, l'acide pantothénique, la biotine, le Glutathion, l'acide ascorbique, le GABA, et l'alpha-tocophérol, les sels de ces molécules, les esters de ces molécules et les anhydrides de ces molécules, sont hydrophobes et la poly-L-lysine, le poly-éthylène-glycol, la poly-ornithine, la poly-arginine ou la poly-histidine sont des polymères hydrophiles.

Ainsi, la composition C2 comprend préférentiellement :
- des conjugués de la taurine, l'acide rétinoïque, la spermine, l'acide pantothénique, la biotine, le Glutathion, l'acide ascorbique, le GABA, et l'alpha-tocophérol, de sels de ces molécules, d'esters de ces molécules et/ou d'anhydrides de ces molécules, avec la poly-lysine, le poly-éthylène-glycol, la poly-ornithine, la poly-arginine ou la poly-histidine, et
- des micelles formées par au moins un de ces conjugués, lesdits micelles encapsulant le co-enzyme Q10, et
- des copolymères de méthionine et de poly-lysine ou poly-éthylène-glycol ou poly-ornithine ou poly-arginine ou poly-histidine, et/ou des copolymère de cystéine et de poly-lysine ou poly-éthylène-glycol ou poly-ornithine ou poly-arginine ou poly-histidine.

Cette configuration permet en particulier :
- d'augmenter la demi-vie des molécules actives de la composition dans l'organisme,
- de cibler les tissus ou les cellules sur lesquelles les molécules de composition doivent agir,
- de permettre aux molécules actives de la composition de passer la barrière hémato encéphalique et de pénétrer dans les cellules
- d'augmenter la stabilité et la biodisponibilité de l'actif.

L'efficacité de la composition C2 est donc plus importante et il est possible d'administrer des doses plus faibles et de réduire la toxicité aigüe ou chronique des molécules actives contenues dans la composition.

Préférentiellement, le ou les polymère(s) conjugués aux molécules ou avec lesquels sont copolymérisés les molécules, sont choisis parmi la poly-L-lysine, le poly-éthylène-glycol, la poly-L-ornithine, la poly-L-arginine et la poly-L-histidine.

Selon un mode de réalisation il s'agit de la poly-lysine, préférentiellement de la poly-L-lysine. Préférentiellement la poly-lysine utilisée est une poly-L-lysine de poids moléculaire compris entre 12 000 et 20 000 Da.

Ainsi, selon une variante, la composition C2 comprend au moins :
- A. les conjugués suivants, chaque conjugué étant constitué par une molécule liée de façon covalente à une poly-lysine :
- un ou plusieurs conjugués Taurine-Poly-L-Lysine
- un ou plusieurs conjugués Spermine-Poly-L-Lysine
- un ou plusieurs conjugués Pantothényl-Poly-L-Lysine
- un ou plusieurs conjugués Alpha tocophérol-Poly-L-Lysine
- un ou plusieurs conjugués Biotinyl-Poly-L-Lysine
- un ou plusieurs conjugués Glutathion-Poly-L-Lysine
- un ou plusieurs conjugués Ascorbyl-Poly-L-Lysine
- un ou plusieurs conjugués GABA-Poly-L-Lysine,
- un ou plusieurs conjugués Rétinoyl-Poly-L-Lysine, et

B. Co-enzyme Q10, et/ou un ester et/ou un sel de Co-enzyme Q10, encapsulées dans des micelles, préférentiellement dans des micelles formées par un ou plusieurs des conjugués listés au point A, et C. des copolymères méthionine-Poly-L-Lysine et des copolymères cystéine-Poly-L-Lysine, la méthionine et/ou la cystéine pouvant être remplacé par un ester ou un sel de ces molécules.

Dans cette composition, la poly-L-Lysine peut être remplacée par une autre poly-lysine ou par le poly-éthylène-glycol, une poly-L-ornithine, une poly-L-arginine ou une poly-L-histidine.

La composition C2 selon l'invention peut se présenter sous forme liquide ou sous forme solide. Lorsqu'elle est sous forme liquide la composition comprend au moins de l'eau et les constituants ciavant mentionnés. La forme solide est préférentiellement obtenue à partir de la forme liquide, de façon préférée par lyophilisation. Ainsi lorsque la composition C2 sous forme liquide comprend des micelles et qu'elle est lyophilisée sous forme solide, les micelles se reforment lorsque la composition C2 sous forme solide est de nouveau mise dans une solution aqueuse.

La composition C2 comprend également préférentiellement des excipients pharmaceutiquement acceptables. Ces excipients peuvent être choisis en particulier pour répondre aux pH et osmolarité de solution injectables chez l'homme ou l'animal. Il peut s'agir par exemple d'acides ou de bases pour ajuster le pH ou de NaCl pour ajuster l'osmolarité.

La composition C2 selon l'invention est destinée à être administrée à l'être humain, ou à un animal et se présente par conséquent sous une forme adaptée à une telle administration. Lorsqu'elle se présente sous forme liquide, elle est préférentiellement adaptée à une administration par voie souscutanée ou par voie intraveineuse, notamment par voie intraveineuse en perfusion, et elle est conditionnée dans des contenants adaptés et connus de l'homme du métier pour conditionner ce type de produits. La composition C2 peut être aussi administrée sous forme liquide via une pompe, du type des pompes à insuline.

Lorsqu'elle se présente sous forme solide, elle est préférentiellement adaptée à une administration :
- par voie transcutanée et elle est préférentiellement formulée et conditionnée sous forme d'un patch, ou
- par voie nasale sous forme de poudre, ou
- par voie sublinguale sous forme de poudre ou de comprimé, ou
- par voie transmucosale et elle est préférentiellement formulée et conditionnée sous forme d'un comprimé muco-adhésif.

### Procédé de fabrication de la composition C2

La composition C2 selon l'invention peut être fabriquée par tout procédé adapté.

Si les molécules qui constituent la composition C2 sont utilisées telles quelles dans un solvant, elles peuvent être mélangées toutes ensemble dans ledit solvant.

Si les molécules dans la composition C2 sont conjuguées à des polymères pour une partie, copolymérisées pour d'autres et dans des micelles pour d'autres, le procédé de fabrication peut comprendre les étapes suivantes :
- a. créer un premix (pré-mélange) amphiphile : mélanger dans une solution aqueuse un ou plusieurs conjugués choisis parmi :
- un ou plusieurs conjugués Taurine-Poly-L-Lysine
- un ou plusieurs conjugués Spermine-Poly-L-Lysine
- un ou plusieurs conjugués Pantothényl-Poly-L-Lysine
- un ou plusieurs conjugués Alpha tocophérol-Poly-L-Lysine
- un ou plusieurs conjugués Biotinyl-Poly-L-Lysine
- un ou plusieurs conjugués Glutathion-Poly-L-Lysine
- un ou plusieurs conjugués Ascorbyl-Poly-L-Lysine
- un ou plusieurs conjugués GABA-Poly-L-Lysine,
- un ou plusieurs conjugués Rétinoyl-Poly-L-Lysine, et
- b. ajouter dans le premix amphiphile au moins du co-enzyme Q10, et mettre sous agitation de façon à former des micelles formées par un ou plusieurs des conjugués du premix amphiphile encapsulant le co-enzyme Q10,
- ajouter des co-polymères de méthionine-Poly-L-Lysine et des copolymères cystéine-Poly-L-Lysine dans le mélange.

En effet, une variante de l'invention consiste à tirer parti de la nature amphiphile de la plupart des composants individuels, pour créer un prémélange amphiphile qui permet la solubilisation contrôlée du co-enzyme Q10 hydrophobe. Le processus de solubilisation selon un mode de réalisation préféré consiste en une addition contrôlée des molécules hydrophobes aux prémélanges amphiphiles et à laisser le temps nécessaire à leur dissolution sous agitation.

La Poly-L-lysine peut être remplacée par une autre poly-lysine ou par le poly-éthylène-glycol, une poly-L-ornithine, une poly-L-arginine ou une poly-L-histidine.

Préférentiellement, l'agitation est réalisée pendant au moins 5 minutes, encore plus préférentiellement entre 5 et 20 minutes, et de façon préférée à une vitesse d'agitation inférieure ou égale à 900 tours par minute, en particulier à une vitesse d'agitation comprise entre 50 et 800 tours par minute.

Selon un mode de réalisation préféré, le procédé de fabrication selon l'invention comprend également une étape d. de séparation des phases soluble et insoluble, pour récupérer la phase soluble. Dans ce cas la phase insoluble est éliminée et la phase soluble constitue la composition C2 selon l'invention. En effet, un processus de séparation physique (filtration, ultrafiltration) est préférentiellement réalisé pour assurer l'isolement de la fraction soluble, contenant à la fois le prémélange amphiphile, les copolymères et les molécules solubilisées.

La composition C2 sous forme liquide peut ensuite être lyophilisée ou déshydratée pour être sous forme solide, préférentiellement par lyophilisation lente par exemple entre 12 et 36 heures.

Par ailleurs si des conjugués molécule active-polymère et/ou les co-polymères de la composition C2 ne sont pas intégrés dans le premix de l'étape a ou c, ceux-ci peuvent être ajoutés dans le mélange après l'étape b. c'est-à-dire après la formation des micelles et l'encapsulation du co-enzyme Q10.

Les conjugués molécule active-polymère peuvent être fabriqués par tout moyen connu de l'homme du métier pour conjuguer une molécule de façon covalente à un polymère en fonction de leur nature chimique. Ainsi :
- l'acide ascorbique est conjugué à un polymère par une liaison carbamate,
- la spermine et la taurine sont conjugués à un polymère par une liaison urée.
- le glutathion, l'acide pantothénique, l'acide aminobutyrique, la biotine, l'alpha tocophérol et le GABA, l'acide rétinoïque sont conjugués à un polymère par une liaison amide.

Des exemples de réalisation de liaisons urée et carbamates sont décrits par exemple dans :
- Zhuxian Z, Jianbin T, Qihang S, William J. A multifunctional PEG-PLL drug conjugate forming redox-responsive nanoparticles for intracellular drug delivery Issue 38, 2015. Journal of Materials Chemistry B (liaisons amide),
- Scheper V, Wolf M, Scholl M, Kadlecova Z, Perrier T, Klok HA, Saulnier P, Lenarz T, Stöver T. Potential novel drug carriers for inner ear treatment: hyperbranched polylysine and lipid nanocapsules. Nanomedicine (Lond). 2009 Aug;4(6):623-35 (liaisons urée),
- Stéphanie Gac-Breton, Jean Coudane, Mahfoud Boustta & Michel Vert (2004) Norfloxacin-Poly(l-Lysine Citramide Imide) Conjugates and Structure-dependence of the Drug Release, Journal of Drug Targeting, 12:5, 297-307, (liaisons carbamate),
- Elmore, W. M. (2013). Nanoparticles Stabilized with MPEG-Polylysine Carbamate: Synthesis and Characterization, (liaisons carbamate),
- Ning-Ping Huang, Janos Vörös, Susan M. De Paul, Marcus Textor, and Nicholas D. Spencer. Biotin-Derivatized Poly(l-lysine)-g-poly(ethylene glycol): A Novel Polymeric Interface for Bioaffinity Sensing. Langmuir 2002 18 (1), 220-230 (liaisons carbamate).

Les copolymères peuvent être fabriqués par tout moyen connu de l'homme du métier. Les molécules de méthionine ou de cystéine sont incorporées dans la structure du polymère préférentiellement en présence d'un solvant anhydre et selon un mode de réalisation dans un rapport de 1 molécule de cystéine ou méthionine pour 10 monomères du polymère.

### Utilisation de la composition C2

La composition C2 est une formulation particulièrement adaptée pour prévenir et/ou traiter les maladies multifactorielles, notamment les maladies chroniques multifactorielles. La composition C2 est particulièrement utile pour la prévention et le traitement des maladies neurodégénératives, auto-immunes, infectieuses ou des cancers. En particulier, elle présente une grande efficacité dans la prévention et le traitement de la maladie de Charcot. La composition C2 intervient sur les processus classiques et connus pour diminuer la chronicité des maladies. Son action est tout particulièrement adaptée aux maladies neurodégénératives, auto-immunes, cancer et infectieuse.

Elle est notamment capable d'agir de façon combinée :
- comme immuno-modulateur et régulateur de l'auto-immunité, en particulier grâce à la présence d'acide rétinoïque et de GABA,
- comme neuroprotecteur, en particulier grâce à la présence de taurine, co-enzyme Q10, GABA
- comme anti-inflammatoire, en particulier grâce à la présence de spermine,
- comme protecteur cellulaire, en particulier grâce à la présence d'acide pantothénique, co-enzyme Q10 (protection en particulier au niveau de la chaine mitochondriale)
- comme protecteur des membranes cellulaires, en particulier grâce à la présence de l'alpha-tocophérol,
- comme anti-oxydant, en particulier grâce à la présence de taurine, de cystéine, de méthionine, spermine, acide pantothénique, alpha-tocophérol, co-enzyme Q10, glutathion, acide ascorbique,
- comme régulateur du métabolisme cellulaire piégeur des dérivés oxygénés, en particulier grâce à l'acide ascorbique,
- comme précurseur du glutathion, en particulier grâce à la présence de cystéine,
- sur la stabilisation des membranes cellulaires,
- comme piégeur de radicaux libres et de métaux, en particulier grâce à la présence de taurine, de cystéine, de méthionine , spermine, acide pantothénique, co-enzyme Q10, glutathion.

Les différentes molécules présentes agissent en synergie et permettent notamment de prévenir ou lutter contre les facteurs à l'origine des maladies chroniques et en particulier de la maladie de Charcot.

Ainsi, la composition C2 peut être utilisée pour prévenir et/ou lutter contre les maladies chroniques notamment pour agir sur les métabolismes spécifiques de la pathologie. La composition C2 peut être utilisée en particulier pour diminuer la chronicité des maladies chroniques.

Préférentiellement, la composition C2 est utilisée en prévention ou en traitement dès les premiers symptômes d'une maladie chronique, en particulier une maladie neurodégénérative et spécifiquement la maladie de Charcot.

L'invention a donc également pour objet une composition C2 selon l'invention pour son utilisation comme médicament, notamment chez l'Homme ou l'animal c'est-à-dire pour un sujet Humain ou animal.

En particulier l'invention a pour objet une composition C2 pour son utilisation dans la prévention et/ou le traitement d'une pathologie multifactorielle, en particulier une pathologie multifactorielle chronique, notamment une pathologie choisie parmi les maladies inflammatoires, les maladies neurodégénératives, les maladies bactériennes, les maladies virales, les maladies auto-immunes, les allergies et intolérances alimentaires, et en particulier la maladie de Charcot.

Selon un mode de réalisation particulier, l'invention a pour objet une composition C1 pour son utilisation dans la prévention et/ou le traitement d'une pathologie choisie parmi l'eczéma, le psoriasis, les maladies inflammatoires chroniques intestinales(notamment maladie de Crohn et Coeliaque), la maladie d'Alzheimer, la maladie de Parkinson, la sclérose en plaque, la maladie de Charcot.

La composition C2 est très préférentiellement utilisée avec la composition C1.

Un objet particulier de l'invention est par conséquent, la composition C1 pour son utilisation avec la composition C2 comme médicament, en particulier dans la prévention et/ou le traitement d'une pathologie multifactorielle, en particulier une pathologie multifactorielle chronique, notamment une pathologie choisie parmi les maladies inflammatoires, les maladies neurodégénératives, les maladies bactériennes, les maladies virales, les maladies auto-immunes, les allergies et intolérances alimentaires, et notamment dans la prévention et/ou le traitement d'une pathologie choisie parmi l'eczéma, le psoriasis, la maladie de Crohn, la maladie cœliaque, les maladies inflammatoires chroniques intestinales, la maladie d'Alzheimer, la maladie de Parkinson, la sclérose en plaque, la maladie de Charcot.

Cette utilisation comme médicament de la composition C1 et de la composition C2 est simultanée ou séquencée dans le temps. En particulier, il est préférable d'administrer la composition C1 d'abord, puis la composition C2, les deux administrations étant préférentiellement séparées d'au moins 1 heure, encore plus préférentiellement d'au moins 4 heures, idéalement une le matin et une le soir.

Les compositions C1 et C2 peuvent également être utilisées pour traiter ou prévenir des maladies associées à une dysbiose pathologique du microbiote intestinal. Notamment les compositions C1 et C2 peuvent être utilisées pour prévenir ou traiter:
- une maladie neurodégénérative associée à une dysbiose pathologique du microbiote intestinal, par exemple une maladie choisie parmi la sclérose latérale amyotrophique, la sclérose en plaque, la maladie de Parkinson, et la maladie d'Alzheimer
- une maladie intestinale associée à une dysbiose pathologique du microbiote intestinal, par exemple une maladie choisie parmi la maladie de Crohn, les maladies inflammatoires chronique de l'intestin, la rectocolite hémorragique, le syndrome de l'intestin irritable, la colite ulcéreuse, la polyarthrite rhumatoïde et l'intolérance alimentaire en particulier l'intolérance au gluten.

L'invention est à présent décrite par des exemples et des résultats d'essais.

### Exemples

### Exemple 1 : composition C1

Un exemple de composition C1 adaptée pour des tests sur des souris est présenté ci-après.

Les quantités suivantes de conjugués ont été pesées selon le tableau 1 pour préparer un stock de 300 ml de composition C1 10x. Tous les conjugués ont été pesés avec une balance analytique dans une hotte à flux laminaire.

**[Tableau 1]**

| Conjugué PLL (PLL = Poly-L-Lysine) | Quantité (mg) |
|---|---|
| Oléyl-PLL | 201 |
| Palmitic-PLL | 78 |
| Lauryl-PLL | 189 |
| Lynoléyl-PLL | 78 |
| Azélayl-PLL | 75 |
| Plamitoleyl-PLL | 156 |
| Thioctyl-PLL | 225 |
| Myristyl-PLL | 78 |
| Orothyl-PLL | 72 |
| Acetate-PLL | 174 |
| Butyrate-PLL | 285 |
| Lactate-PLL | 177 |
| Propionate-PLL | 177 |

Chaque conjugué a été pesé dans un tube à centrifuger stérile de 50 ml, et a été dissous dans 30 ml d'eau, à l'aide d'un vortex. Chaque conjugué a été agité pendant une durée comprise entre 10 et 20 minutes en fonction de la solubilité du conjugué.

En parallèle des molécules de cholestérol et de farnésylcystéine ont été dissous séparément à la concentration approximative de 50 mg/ml dans de l'éthanol absolu et filtrés sur un filtre filtrant de 0,22 µm. Le solvant a été évaporé par un rotavaporateur. Les quantités nécessaires de solides secs ont été pesées dans une hotte à flux laminaire, telles que présentées dans le tableau 2.

**[Tableau 2]**

| Molécule | Quantité (mg) |
|---|---|
| Cholestérol | 12 |
| Farnésylcystéine | 12 |

Dans un flacon stérile de 500 ml et muni d'une barre magnétique, 23,8 ml de chaque solution conjuguée précédemment préparée ont été ajoutés sous agitation magnétique (700 tours par minute).

Les quantités correspondantes de sels (préalablement lyophilisés) à 300 de PBS ont été ajoutées à la solution de conjugués sous agitation magnétique (700 tours par minute).

Le cholestérol et la farnésylcystéine ont été ajoutés et le mélange et laissés sous agitation pendant 25 heures (700 tours par minute).
30 ml de formulation C1 10 x ont été prélevés dans une hotte à flux laminaire et ajoutés à un nouveau flacon stérile de 500 ml.
270 ml de PBS stérile ont été ajoutés afin d'obtenir la formulation C1 1x. La formulation a été agitée pendant 30 minutes.

Les flacons ont été préparés selon le plan suivant en utilisant une pipette stérile et calibrée de 5 ml :
C1 1X = 83 flacons, 2 ml chacun.
C1 10X = 83 flacons de 2 ml chacun.

Les 166 flacons ont été placés dans un bac de lyophilisation en acier et un cycle de lyophilisation d'une journée a été lancé.

### Exemple 2 : composition C2

Un exemple de composition C2 adaptée pour des tests sur souris est présenté ci-après.

Les quantités suivantes de conjugués ont été pesées selon le tableau 3 pour préparer un stock de 300 ml de composition C2 10x. Tous les conjugués ont été pesés avec une balance analytique dans une hotte à flux laminaire.

**[Tableau 3]**

| Conjugué PLL (PLL = Poly-L-Lysine) | Quantité (mg) |
|---|---|
| Alpha-tocophérol-PLL | 210 |
| Ascorbyl-PLL | 189 |
| Biotinyl-PLL | 204 |
| GABA-PLL | 378 |
| Glutathion-PLL | 222 |
| Panthotényl-PLL | 204 |
| Retynoil-PLL | 606 |
| Spermine-PLL | 198 |
| Taurine-PLL | 330 |
| PLL/Cystéine (copolymère) | 180 |
| PLL/Méthionine (copolymère) | 303 |

Chaque conjugué a été pesé dans un tube à centrifuger stérile de 50 ml, et a été conservé au congélateur à -20°C jusqu'à son utilisation.

En parallèle le coenzymeQ10 a été dissous à la concentration approximative de 50 mg/ml dans de l'éthanol absolu et filtré à travers un filtre de 0,22 µm. Le solvant a été évaporé par un rotavaporateur. Les quantités nécessaires de solides secs ont été pesées dans une hotte à flux laminaire, telles que présentées dans le tableau 4.

**[Tableau 4]**

| Molécule | Quantité (mg) |
|---|---|
| Co-enzyme Q10 | 156 |

Le solide a été conservé dans un tube à centrifuger stérile à -20°C jusqu'à son utilisation.

Les tubes de centrifugation de 50 ml précédemment préparés avec les conjugués et copolymères ont été pris et laissés se réchauffer à température ambiante. Après ce temps, 30 ml d'eau ont été ajoutés à chaque tube de centrifugation.

Les conjugués et copolymères ont été dissous à l'aide d'un vortex, en agitant pendant un temps compris entre 10 et 20 minutes, selon la solubilité des conjugués et copolymères.

Le tube de centrifugation contenant la coenzyme Q10 a également été sorti du congélateur et laissé se réchauffer à température ambiante.

Un flacon stérile de 500 ml a été pris et muni d'une barre magnétique. 27,3 ml de chaque solution conjuguée précédemment préparée ont été ajoutés au flacon sous agitation magnétique (700 tours par minute).

Les quantités correspondantes de sels (préalablement lyophilisés) à 300 de PBS ont été ajoutées à la solution de conjugués sous agitation magnétique (700 tours par minute).

Le coenzymeQ10 a été ajouté et le mélange a été laissé sous agitation pendant 2 heures (700 tours par minute).

Après 2 heures, 30 ml de formulation C2 10 x ont été prélevés dans une hotte à flux laminaire et ajoutés à un nouveau flacon stérile de 500 ml.

270 ml de PBS stérile ont été ajoutés afin d'obtenir la formulation C2 x1. La formulation a été agitée pendant 30 minutes.

Les flacons ont été préparés selon le plan suivant en utilisant une pipette stérile et calibrée de 5 ml :
DP2 1X = 83 flacons, 2 ml chacun.
DP2 10X = 83 flacons de 2 ml chacun.

Les 166 flacons ont été placés dans le bac de lyophilisation en acier et un cycle de lyophilisation d'une journée a été lancé.

### Exemple 3 : composition C1

Un exemple de composition C1 adaptée pour des tests sur des rats est présenté ci-après.

Le procédé de fabrication de la composition est similaire à celui présenté à l'exemple 1. Les concentrations de chacun des constituants sont différentes et présentés dans le tableau 5.

**[Tableau 5]**

| Composés | Concentration C1 10X (mg/mL) | Concentration C1 1X (mg/mL) |
|---|---|---|
| Acetate PLL | 1,16 | 0,12 |
| Azélayl-PLL | 0,50 | 0,05 |
| Butyrate-PLL | 1,88 | 0,19 |
| Lauryl-PLL | 1,26 | 0,13 |
| Lactate-PLL | 1,18 | 0,12 |
| Linoléyl-PLL | 0,52 | 0,05 |
| Myristyl-PLL | 0,51 | 0,05 |
| Oléyl-PLL | 1,33 | 0,13 |
| Orotyl-PLL | 0,48 | 0,05 |
| Palmitic-PLL | 0,52 | 0,05 |
| Palmitoléyl-PLL | 1,04 | 0,10 |
| Propionate-PLL | 1,17 | 0,12 |
| Thioctyl-PLL | 1,49 | 0,15 |
| Cholesterol | 0,09 | 0,01 |
| FarCys | 0,07 | 0,01 |

La somme des concentrations en conjugués PLL + cholestérol + Farnésylcystéine pour C1 10X est de 13,2 mg/mL.

La somme des concentrations des conjugués PLL + cholestérol + Farnésylcystéine pour C1 1X est de 1,32 mg/ml.

### Exemple 4 : composition C1

Un exemple de composition C1 adaptée pour l'Homme est présenté ci-après.

Le procédé de fabrication de la composition est similaire à celui présenté à l'exemple 1. Les concentrations de chacun des constituants sont différentes et présentés dans le tableau 6.

**[Tableau 6]**

| Composés | Concentration C1 10X (mg/mL) | Concentration C1 1X (mg/mL) |
|---|---|---|
| Acetate PLL | 7,192 | 0,744 |
| Azélayl-PLL | 3,1 | 0,31 |
| Butyrate-PLL | 11,656 | 1,178 |
| Lauryl-PLL | 7,812 | 0,806 |
| Lactate-PLL | 7,316 | 0,744 |
| Linoléyl-PLL | 3,224 | 0,31 |
| Myristyl-PLL | 3,162 | 0,31 |
| Oléyl-PLL | 8,246 | 0,806 |
| Orotyl-PLL | 2,976 | 0,31 |
| Palmitic-PLL | 3,224 | 0,31 |
| Palmitoléyl-PLL | 6,448 | 0,62 |
| Propionate-PLL | 7,254 | 0,744 |
| Thioctyl-PLL | 9,238 | 0,93 |
| Cholesterol | 0,558 | 0,062 |
| FarCys | 7,192 | 0,744 |

### Exemple 5 : composition C2

Un exemple de composition C2 adaptée pour l'Homme est présenté ci-après.

Le procédé de fabrication de la composition est similaire à celui présenté à l'exemple 2. Les concentrations de chacun des constituants sont différentes et présentés dans le tableau 7.

**[Tableau 7]**

| Composés | Concentration C1 10X (mg/mL) | Concentration C1 1X (mg/mL) |
|---|---|---|
| Alpha-tocophérol-PLL | 8,61 | 0,861 |
| Ascorbyl-PLL | 7,749 | 0,7749 |
| Biotinyl-PLL | 8,364 | 0,8364 |
| GABA-PLL | 15,498 | 1,5498 |
| Glutathion-PLL | 9,102 | 0,9102 |
| Panthotényl-PLL | 8,364 | 0,8364 |
| Retynoil-PLL | 24,846 | 2,4846 |
| Spermine-PLL | 8,118 | 0,8118 |
| Taurine-PLL | 13,53 | 1,353 |
| PLL/Cystéine (copolymère) | 7,38 | 0,738 |
| PLL/Méthionine (copolymère) | 12,423 | 1,2423 |
| Coenzyme Q10 | 6,396 | 0,6396 |

### Résultats d'essais

### Etude de l'effet de la composition C1 selon l'invention pour restaurer la perméabilité intestinale

Le but de cette étude est d'évaluer l'efficacité d'une formulation injectable selon l'invention sur la restauration de la perméabilité intestinale suite à l'induction d'une inflammation intestinale induite par du dextran sulfate de sodium (DSS) chez le rat.

L'épithélium intestinal est une barrière dynamique qui limite l'accès des bactéries, des parasites, des virus mais aussi de certaines molécules chimiques aux tissus de l'hôte. Il est composé de différents types de cellules ayant chacune un rôle bien défini, tels que les entérocytes responsables de la fonction d'absorption des nutriments, les cellules caliciformes sécrétant le mucus dont l'épaisseur maintient les bactéries et autres microorganismes à distance des entérocytes, des cellules de Paneth, situées au fond des cryptes de l'intestin grêle, participant au maintien de l'homéostasie et au rôle de défense de la muqueuse intestinale via la sécrétion d'agents antimicrobiens de type défensines et les cellules microfold dites cellules M, spécialisées dans l'endocytose d'antigènes, de molécules ou encore de microorganismes présents dans le lumen intestinale. Elles jouent le rôle de cellules présentatrices d'antigène puisqu'elles présentent au système immunitaire sous-jacent les antigènes endocytés. La barrière physique épithéliale assurée par les entérocytes est renforcée par une couche de glycocalyx et de mucus épaisse (couche plus fine au niveau des cellules M pour accéder plus facilement au microbiome du lumen intestinal) et la sécrétion IgA par transcytose entérocytaire produits par les plasmocytes sous-jacents. Les cellules épithéliales forment une monocouche de cellules polarisées reposant sur la lamina propria via leur pôle basolatéral et étroitement liées entre elles par des jonctions serrées. Dans cette lamina propria, se situe les plaques de Peyer, constituées de follicules riches en lymphocytes, site initiateur de des réponses pro ou anti-inflammatoires. De nombreuses pathologies sont associées à une altération de la perméabilité intestinale telles que les maladies inflammatoires chroniques de l'intestin, l'obésité, la sclérose en plaques, la maladie de Charcot et Alzheimer. Cette altération conduit à une augmentation de la perméabilité intestinale aboutissant le plus souvent par une inflammation de la paroi de l'intestin.

La composition testée dans cette étude est la composition C1 de l'exemple 3.

L'épithélium intestinale est une barrière dynamique qui limite l'accès des bactéries, des parasites, des virus mais aussi de certaines molécules chimiques aux tissus de l'hôte. Il est composé de différents types de cellules ayant chacune un rôle bien défini, tels que les entérocytes responsables de la fonction d'absorption de l'intestin, les cellules caliciformes sécrétant le mucus, les cellules M responsables du passage de macromolécules et des Antigènes de la lumière intestinale (Lamina Propria) vers les cellules immunitaires sous-jacentes ainsi que des cellules neuroendocrines. La barrière physique épithéliale assurée par les entérocytes est renforcée par une couche de glycocalyx et de mucus épaisse. Les cellules épithéliales forment une monocouche de cellules polarisées reposant sur la lamina propria via leur pôle basolatéral et étroitement liées entre elles par des jonctions serrées. De nombreuses pathologies sont associées à une altération de la perméabilité intestinale telles que les maladies inflammatoires chroniques de l'intestin, l'obésité, la sclérose en plaques, la maladie de Charcot et Alzheimer. Cette altération conduit à une augmentation de la perméabilité intestinale aboutissant le plus souvent par une inflammation de la paroi de l'intestin.

L'objectif principal de l'étude est de mettre en évidence l'efficacité de l'invention sur la remédiation de la perméabilité intestinale suite à l'induction d'une inflammation intestinale chronique.

Pour tester ces hypothèses, des rats ont reçu différentes doses de la composition C1 de l'exemple 3, administrées par injection sous cutanée après induction de l'inflammation intestinale.

Le protocole opératoire est décrit en suivant.

### 1- Mise en place du modèle d'inflammation chronique

Après leur arrivée, les animaux (rats Wistar, 280-300g) ont été élevés dans des cages de 900 cm² (3 animaux par cages). Durant cette période d'acclimatation, les animaux ont eu libre accès à l'eau et la nourriture. Après cette période, les animaux (n = 9) ont reçu ad libitum la nourriture et une solution de 2% (m/v) de Dextran Sulfate de Sodium (DSS) dans l'eau de boisson. L'induction de l'inflammation intestinale a été suivie 3, 5 et 7 jours après le début de l'ajout de DSS. Un groupe témoin (n = 3) a reçu de la nourriture et de l'eau ad libitum. Si des signes de douleur sont observés, l'animal concerné a été isolé et du paracétamol (200 mg/kg) lui a été administré par gavage 2 fois par jours. Ces animaux ont été sortis du schéma expérimental. Si une perte de poids importante a été observée, les animaux ont été sacrifiés lorsqu'ils avaient atteint 80% de leur poids initial. Enfin, les animaux ont été sacrifiés s'ils présentaient une attitude inhabituelle traduisant un mal-être.

Les conditions d'hébergement ont été les suivantes : température 20-24°C, hygrométrie 60 plus ou moins 10%, cycle 12h/12h.

Les animaux ont été observés quotidiennement pour repérer des signes de stress ou de douleur. Des pesées journalières des animaux, de la nourriture et de l'eau ont été réalisées. L'apparition des signes cliniques de l'inflammation est basée sur la perte de poids corporel, la consistance des selles (normale-0, molle-2, diarrhée-4) et les saignements rectaux (0 - 4). Les résultats ont permis de déterminer un index de sévérité de l'inflammation qui représente la moyenne des scores obtenus pour la consistance des selles et des saignements rectaux. La perte de poids représente le pourcentage de différence de poids des rats à J1 (ajout DSS) à J7.

### 2- Détermination de la dose « efficace »

Après leur arrivée, les animaux (rats Wistar, 280-300g) ont été élevés dans des cages de 900 cm² (5 animaux par cages). Durant cette période d'acclimatation, les animaux ont eu libre accès à l'eau et la nourriture. Après cette période, les animaux (n = 30) ont été répartis en 4 groupes (Témoin - n = 5, DSS + composition C1 10X - n = 10). Le groupe « Témoin » a reçu de la nourriture et de l'eau ad libitum durant toute l'expérimentation. Les rats ont reçu de la nourriture et une solution de DSS de 2% dans l'eau de boisson ad libitum pendant 3, 5 ou 7 jours (durée déterminée lors de la première expérimentation). Après l'induction de l'inflammation intestinale chaque rat a reçu une injection intra-péritonéale de 1mL de composition C1. Les administrations de composition C1 ont été réalisées en 1 fois par jour. Si des signes de douleur ont été observés, l'animal concerné a été isolé et du paracétamol (200 mg/kg) lui a été administré par gavage 2 fois par jours. Ces animaux ont été sortis du schéma expérimental. Si une perte de poids importante a été observée, les animaux ont été sacrifiés lorsqu'ils ont atteint 80% de leur poids initial. Enfin, les animaux ont été sacrifiés s'ils présentaient une attitude inhabituelle traduisant un mal-être.

Les conditions d'hébergement ont été les suivantes : température 20-24°C, hygrométrie 60 plus ou moins 10%, cycle 12h/12h.

Les animaux ont été observés quotidiennement pour repérer des signes de stress ou de douleur. Des pesées journalières des animaux, de la nourriture et de l'eau ont été réalisées.

L'apparition des signes cliniques de l'inflammation est basée sur la perte de poids corporel, la consistance des selles (normale-0, molle-2, diarrhée-4) et les saignements rectaux (0 - 4).

Les résultats permettent de déterminer un index de sévérité de l'inflammation qui représente la moyenne des scores obtenus pour la consistance des selles et des saignements rectaux. La perte de poids représente le pourcentage de différence de poids des rats ((JX x J0)/J0)*100). A la fin de l'expérimentation, les animaux ont été anesthésiés (cf. prélèvement organes) et différents prélèvements ont été réalisés : sang, intestin grêle, cæcum, côlon, foie, rate et plusieurs paramètres évalués : taille du cæcum, poids du foie, de la rate, et du cæcum.

### Etude de perméabilité

Une étude de perméabilité a été réalisée sur organe isolé (jéjunum ou côlon) pour déterminer l'efficacité de la dose de composition C1 sur le rétablissement de la perméabilité intestinale.

### Prélèvement des fragments digestifs

Cette approche ex vivo utilise un fragment de jéjunum ou de côlon issu du tractus digestif de rat. Les rats ont été anesthésiés par inhalation d'isoflurane (1000mg/g) avec une induction à 3% et un entretien au cours de l'opération de 1,5%. Après laparotomie et ligature de l'artère cœliaque accolée à l'œsophage, un fragment de 10 cm de jéjunum ou de côlon a été prélevé. Le prélèvement a été pesé. Les rats ont été ensuite sacrifiés par injection de pentobarbital sodique (12 mg/100g).

### Préparation de l'organe éversé et prélèvement

Les compartiments séreux et muqueux de l'organe ont été rapidement rincés avec du sérum physiologique (37°C) et l'organe a été éversé. Une ligature a été réalisée à l'une des extrémités. La seconde extrémité permet l'introduction de 1 mL de milieu Krebs-Henseleit. Cette extrémité a ensuite été ligaturée, l'organe a été pesé puis placé dans une cuve contenant 10 mL de milieu de survie Krebs-Henseleit additionnés à 250 mg de FITC-dextran de 4 Kda (compartiment muqueux). Durant toute l'expérimentation, les organes éversés ont été maintenus à 37°C et oxygénés avec un mélange O2/CO2 (95%/5%). Toutes les 30 minutes, le milieu du compartiment muqueux a été agité en réalisant 3 cycles d'aspiration-refoulement à l'aide d'une micropipette de 1mL. Après 2 h d'incubation, un prélèvement de 1 mL a été réalisé côté muqueux.

Les organes ont été retirés de la cuve en verre et une des extrémités a été coupée. Le milieu séreux a été prélevé dans un tube préalablement pesé. Après prélèvement, le tube a été à nouveau pesé afin d'évaluer le transfert de milieu de survie au cours de l'expérimentation. Après la pesée, le FITC-Dextran a été dosé par fluorométrie en duplicat (excitation, 490 nm ; émission, 530 nm).

Après avoir pesé l'organe, une coupe longitudinale de ce dernier a été réalisée et ce dernier a été rapidement congelé dans de l'azote liquide. L'organe congelé a été ensuite introduit dans un sachet zip et a été congelé dans l'azote liquide puis conservé à -80°C.

### Etude des paramètres de l'inflammation

L'étude a été faite par le comportement des animaux traités. En effet une inflammation provoque une agitation exacerbée chez les animaux.

### Résultats

Les résultats sur l'observation des symptômes de l'inflammation sont présentés dans les tableaux 8 à 10 (avant injection de la composition C1) et sur les tableaux 11 à 15 (après injection de la composition C1). Sur ces tableaux :
- la variation de poids en pourcent est calculé comme suit : ((JX x J0)/J0)*100)
- La consistance des selles est évaluée comme suit : 0 = normale ; 2 = molle ; 3 = diarrhée
- Les saignements rectaux sont évalués comme suit : 0 = rien ; 1 = légers ; 2 = modérés ; 3= importants ; 4 = abondants.

**[Tableau 8]**

| | Jours | J3 | | | J4 | | |
|---|---|---|---|---|---|---|---|
| | | Variation du poids (%) | Consistance des selles (0/2/4) | Saignement (0 - 4) | Variation du poids (%) | Consistance des selles (0 / 2/4) | Saignement (0 - 4) |
| Témoin | Rat 1 | 10,7 | 0 | 0 | 12,14 | 0 | 0 |
| | Rat 2 | 11,1 | 0 | 0 | 13,02 | 0 | 0 |
| | Rat 3 | 9,5 | 0 | 0 | 16,07 | 0 | 0 |
| | Moyenne | 10,4 | 0 | 0 | 13,7 | 0 | 0 |
| DSS 2% | Rat 4 | 5,2 | 0 | 0 | 8,52 | 0 | 0 |
| | Rat 5 | 8,7 | 0 | 0 | 11,00 | 0 | 0 |
| | Rat 6 | 4,8 | 0 | 0 | 9,52 | 0 | 0 |
| | Moyenne | 6,2 | 0 | 0 | 9,7 | 0 | 0 |
| DSS4% | Rat 7 | 7,1 | 2 | 0 | 7,12 | 2 | 0 |
| | Rat 8 | 3,6 | 2 | 0 | 5,57 | 2 | 0 |
| | Rat 9 | 6,2 | 2 | 0 | 6,49 | 2 | 0 |
| | Moyenne | 5,6 | 2 | 0 | 6,4 | 2 | 0 |

**[Tableau 9]**

| | Jours | J5 | | | J6 | | |
|---|---|---|---|---|---|---|---|
| | | Variation du poids (%) | Consistance des selles (0/2/4) | Saignement (0 - 4) | Variation du poids (%) | Consistance des selles (0 / 2/4) | Saignement (0 - 4) |
| Témoin | Rat 1 | 11,07 | 0 | 0 | 14,04 | 0 | 0 |
| | Rat 2 | 13,65 | 0 | 0 | 16,51 | 0 | 0 |
| | Rat 3 | 14,75 | 0 | 0 | 17,74 | 0 | 0 |
| | Moyenne | 13,2 | 0 | 0 | 16,1 | 0 | 0 |
| DSS 2% | Rat 4 | 8,52 | 0 | 0 | 10,03 | 0 | 0 |
| | Rat 5 | 10,33 | 0 | 0 | 15,07 | 0 | 0 |
| | Rat 6 | 7,94 | 0 | 0 | 10,54 | 0 | 0 |
| | Moyenne | 8,9 | 0 | 0 | 11,9 | 0 | 0 |
| DSS4% | Rat 7 | 6,44 | 2 | 0 | 8,14 | 2 | 0 |
| | Rat 8 | 8,20 | 2 | 0 | 9,64 | 2 | 0 |
| | Rat 9 | 5,84 | 2 | 0 | 7,40 | 2 | 0 |
| | Moyenne | 6,8 | 2 | 0 | 8,4 | 2 | 0 |

**[Tableau 10]**

| | Jours | J7 | | |
|---|---|---|---|---|
| | | Variation du poids (%) | Consistance des selles (0 / 2 / 4) | Saignement (0 - 4) |
| Témoin | Rat 1 | 7,25 | 0 | 0 |
| | Rat 2 | 9,46 | 0 | 0 |
| | Rat 3 | 9,31 | 0 | 0 |
| | Moyenne | 8,67 | 0 | 0 |
| DSS 2% | Rat 4 | 4,59 | 2 | 0 |
| | Rat 5 | 6,50 | 2 | 0 |
| | Rat 6 | 4,06 | 2 | 0 |
| | Moyenne | 5,05 | 2 | 0 |
| DSS4% | Rat 7 | 5,08 | 2 | 0 |
| | Rat 8 | 4,62 | 2 | 0 |
| | Rat 9 | 3,90 | 2 | 2 |
| | Moyenne | **4,53** | **2** | **1** |

**[Tableau 11]**

| | Jours | J8 | | | J9 | | |
|---|---|---|---|---|---|---|---|
| | | Variation du poids (%) | Consistance des selles (0 / 2 / 4) | Saignement (0 - 4) | Variation du poids (%) | Consistance des selles (0 / 2 / 4) | Saignement (0 - 4) |
| Témoin | Rat 1 Moyenne | 9,75 | 0 | 0 | 10,64 | 0 | 0 |
| | | 6,53 | 2 | 0 | 3,13 | 2 | 0 |
| DSS 2% | Rat 5 | 4,22 | 2 | 0 | 2,48 | 2 | 0 |
| | Rat 6 | 5,38 | 2 | 0 | 2,80 | 2 | 0 |
| | Moyenne | 6,92 | 4 | 0 | 3,67 | 4 | 0 |
| DSS4% | Rat 8 | 1,36 | 4 | 2 | -1,10 | 4 | 4 |
| | Rat 9 | 4,14 | 4 | 1 | 1,28 | 4 | 2 |
| | Moyenne | 9,75 | 0 | 0 | 10,64 | 0 | 0 |

**[Tableau 12]**

| | Jours | J10 | | | J11 | | |
|---|---|---|---|---|---|---|---|
| | | Variation du poids (%) | Consistance des selles (0 / 2 / 4) | Saignement (0 - 4) | Variation du poids (%) | Consistance des selles (0 / 2 / 4) | Saignement (0 - 4) |
| Témoin | Rat 1 Moyenne | 18,64 | 0 | 0 | 20,11 | 0 | 0 |
| | | 3,17 | 2 | 0 | 5,87 | 2 | 0 |
| DSS 2% | Rat 5 | 5,21 | 2 | 0 | 3,27 | 2 | 0 |
| | Rat 6 | 4,19 | 2 | 0 | 4,57 | 2 | 0 |
| | Moyenne | 4,00 | 4 | 0 | 4,43 | 4 | 0 |
| DSS4% | Rat 8 | -0,88 | 4 | 4 | -0,36 | 4 | 0 |
| | Rat 9 | 1,56 | 4 | 2 | 2,03 | 4 | 0 |
| | Moyenne | 18,64 | 0 | 0 | 20,11 | 0 | 0 |

**[Tableau 13]**

| | Jours | J12 | | | J13 | | |
|---|---|---|---|---|---|---|---|
| | | Variation du poids (%) | Consistance des selles (0 / 2 / 4) | Saignement (0 - 4) | Variation du poids (%) | Consistance des selles (0 / 2 / 4) | Saignement (0 - 4) |
| Témoin | Rat 1 Moyenne | 17,50 | 0 | 0 | 20,18 | 0 | 0 |
| | | 3,73 | 2 | 0 | 3,63 | 2 | 0 |
| DSS 2% | Rat 5 | 0,00 | 2 | 0 | -2,35 | 2 | 0 |
| | Rat 6 | 1,87 | 2 | 0 | 0,64 | 2 | 0 |
| | Moyenne | 4,26 | 4 | 0 | 3,44 | 4 | 0 |
| DSS4% | Rat 8 | -1,30 | 4 | 0 | -2,18 | 4 | 0 |
| | Rat 9 | 1,48 | 4 | 0 | 0,63 | 4 | 0 |
| | Moyenne | 17,50 | 0 | 0 | 20,18 | 0 | 0 |

**[Tableau 14]**

| | Jours | J14 | | | J15 | | |
|---|---|---|---|---|---|---|---|
| | | Variation du poids (%) | Consistance des selles (0 / 2 / 4) | Saignement (0 - 4) | Variation du poids (%) | Consistance des selles (0 / 2 / 4) | Saignement (0 - 4) |
| Témoin | Rat 1 Moyenne | 23,79 | 0 | 0 | 22,89 | 0 | 0 |
| | | 6,53 | 0 | 0 | 2,87 | 0 | 0 |
| DSS 2% | Rat 5 | 4,22 | 0 | 0 | -2,41 | 0 | 0 |
| | Rat 6 | 5,38 | 0 | 0 | 0,23 | 0 | 0 |
| | Moyenne | 6,92 | 4 | 0 | 2,66 | 4 | 2 |
| DSS4% | Rat 8 | 1,36 | 4 | 2 | -2,31 | 4 | 2 |
| | Rat 9 | 4,14 | 4 | 1 | 0,18 | 4 | 2 |
| | Moyenne | 23,79 | 0 | 0 | 22,89 | 0 | 0 |

**[Tableau 15]**

| | Jours | J16 | | | J17 | | |
|---|---|---|---|---|---|---|---|
| | | Variation du poids (%) | Consistance des selles (0 / 2 / 4) | Saignement (0 - 4) | Variation du poids (%) | Consistance des selles (0 / 2 / 4) | Saignement (0 - 4) |
| Témoin | Rat 1 Moyenne | 24,57 | 0 | 0 | 19,64 | 0 | 0 |
| | | 3,20 | 0 | 0 | 0,93 | 0 | 0 |
| DSS 2% | Rat 5 | -3,02 | 0 | 0 | -5,02 | 0 | 0 |
| | Rat 6 | 0,09 | 0 | 0 | -2,04 | 0 | 0 |
| | Moyenne | 2,46 | 2 | 2 | -0,75 | 2 | 2 |
| DSS4% | Rat 8 | -3,31 | 2 | 2 | -2,95 | 2 | 2 |
| | Rat 9 | -0,43 | 2 | 2 | -1,85 | 2 | 2 |
| | Moyenne | 24,57 | 0 | 0 | 19,64 | 0 | 0 |

On constate que la variation de poids a été stabilisée à J12 pour les rats avec DSS 2% et DSS4%.

Concernant la consistance des selles, elle redevient normale après 6 jours de traitement avec la composition selon l'invention pour les rats DSS 2% et après 8 jours de traitement avec la composition selon l'invention pour les rates DSS 4%.

Concernant les saignements rectaux, ils sont absents des rats DSS 2% ce qui signifie qu'il y a eu un rétablissement de la fonction intestinale.

Concernant l'analyse de la perméabilité de la membrane intestinale, les résultats des tests in vivo 10 jours après l'injection de la composition C1 aux rats DSS 2%, par mesure du FITC-dextran, sont présentés dans le tableau 16.

**[Tableau 16]**

| | Témoin (FITC) - Rat 1 | DSS 2% FITC (Rat 5) | DSS 2% FITC (Rat 6) |
|---|---|---|---|
| Plasma | 0,6519 | 0,6024 | 0,7431 |

On constate que le dosage du FITC Dextran 4KDa dans le plasma permet de bien discriminer l'impact de la composition C1 selon l'invention sur les rats ayant reçu 2% de DSS, la fluorescence retrouvée dans le sang est identique à celle du témoin.

Concernant l'analyse de la perméabilité de la membrane intestinale, les résultats des tests ex vivo 10 jours après l'injection de la composition C1 aux rats DSS 2%, sur organes isolés (la perméabilité sur organes isolés a été réalisée sur un fragment de jéjunum distal (5 cm) et sur l'iléon (5 cm) par mesure du FITC-dextran, sont présentés dans le tableau 17.

**[Tableau 17]**

| Organes isolés | 30 min | 60 min | 90 min | 120 min | 150 min |
|---|---|---|---|---|---|
| Témoin (FITC) - Rat 1 | 34,4 | 103,6 | 254,6 | 526,9 | 861,5 |
| DSS 2% FITC (Rat 5) | 30,7 | 137,9 | 457,2 | 841,4 | 1101,0 |
| DSS 2% FITC (Rat 6) | 27,4 | 85,3 | 293,0 | 659,6 | 1030,0 |

On constate que pour les rats traités avec la composition C1 selon l'invention ayant reçu 2% de DSS, les valeurs sont proches de celles observées chez le témoin.

### Analyse Moléculaire du Microbiote Fécal de souris (Sauvage - WT, SOD1, SOD1 + composition selon l'invention)

La souris SOD1 est un modèle animal de dégénérescence axonale constitué par les souris transgéniques exprimant la forme mutée du gène humain de la super oxyde dismutase. Il s'agit du modèle in vivo de référence pour étudier la sclérose latérale amyotrophique.

### Préparation des compositions :

Les quantités suivantes de conjugués ont été pesées pour préparer un stock de 300 ml de composition C1. Tous les conjugués ont été pesés avec une balance analytique dans une hotte à flux laminaire.

**[Tableau 18]**

| Conjugué PLL (PLL = Poly-L-Lysine) | Concentration 1X (mg/mL) | Concentration 10X (mg/mL) |
|---|---|---|
| Oléyl-PLL | 0,063 | 0,63 |
| Palmitic-PLL | 0,024 | 0,24 |
| Lauryl-PLL | 0,059 | 0,59 |
| Linoléyl-PLL | 0,025 | 0,25 |
| Azélayl-PLL | 0,024 | 0,24 |
| Palmitoleyl-PLL | 0,049 | 0,49 |
| Thioctyl-PLL | 0,07 | 0,7 |
| Myristyl-PLL | 0,024 | 0,24 |
| Orothyl-PLL | 0,023 | 0,23 |
| Acetate-PLL | 0,055 | 0,55 |
| Butyrate-PLL | 0,089 | 0,89 |
| Lactate-PLL | 0,056 | 0,56 |
| Propionate-PLL | 0,055 | 0,55 |

Chaque conjugué a été pesé dans un tube à centrifuger stérile de 50 ml, et a été dissous dans 30 ml d'eau, à l'aide d'un vortex. Chaque conjugué a été agité pendant une durée comprise entre 10 et 20 minutes en fonction de la solubilité du conjugué.

En parallèle des molécules de cholestérol et de farnésylcystéine ont été dissoutes séparément à la concentration approximative de 50 mg/ml dans de l'éthanol absolu et filtrés sur un filtre filtrant de 0,22 µm. Le solvant a été évaporé par un rotavaporateur. Les quantités nécessaires de solides secs ont été pesées dans une hotte à flux laminaire.

**[Tableau 19]**

| Molécule | Concentration 1X (mg/mL) | Concentration 10X (mg/mL) |
|---|---|---|
| Cholestérol-PLL | 0,004 | 0,04 |
| Farnésylcystéine-PLL | 0,004 | 0,04 |

Dans un flacon stérile de 500 ml et muni d'une barre magnétique, 23,8 ml de chaque solution conjuguée précédemment préparée ont été ajoutés sous agitation magnétique (700 tours par minute). Les quantités correspondantes de sels (préalablement lyophilisés) à 300 de PBS ont été ajoutées à la solution de conjugués sous agitation magnétique (700 tours par minute).

Le cholestérol et la farnésylcystéine ont été ajoutés et le mélange et laissés sous agitation pendant 25 heures (700 tours par minute).
30 ml de formulation C1 10 x ont été prélevés dans une hotte à flux laminaire et ajoutés à un nouveau flacon stérile de 500 ml.
270 ml de PBS stérile ont été ajoutés afin d'obtenir la formulation C1 1x. La formulation a été agitée pendant 30 minutes.

Les flacons ont été préparés selon le plan suivant en utilisant une pipette stérile et calibrée de 5 ml:
C1 1X = 83 flacons, 2 ml chacun.
C1 10X = 83 flacons de 2 ml chacun.

Les 166 flacons ont été placés dans un bac de lyophilisation en acier et un cycle de lyophilisation d'une journée a été lancé.

Le tableau 20 décrit la composition de la composition C2 utilisée dans cet essai.

**[Tableau 20]**

| Conjugué PLL (PLL = Poly-L-Lysine) | Concentration 1X (mg/mL) | Concentration 10X (mg/mL) |
|---|---|---|
| Alpha-tocophérol-PLL | 0,070 | 0,70 |
| Ascorbyl-PLL | 0,063 | 0,63 |
| Biotinyl-PLL | 0,068 | 0,68 |
| GABA-PLL | 0,126 | 1,26 |
| Glutathion-PLL | 0,074 | 0,74 |
| Panthotényl-PLL | 0,068 | 0,68 |
| Retynoil-PLL | 0,202 | 2,02 |
| Spermine-PLL | 0,066 | 0,66 |
| Taurine-PLL | 0,110 | 1,10 |
| PLL/Cystéine (copolymère) | 0,06 | 0,6 |
| PLL/Méthionine (copolymère) | 0,101 | 1,01 |

Dans cet essai, les composition C1 (décrite au tableau 18 et 19) et C2 (décrite au tableau 20) ont été injectées l'une après l'autre avec une 1 heure d'intervalle en commençant par la composition C1.

Les fèces de cinq souris femelles de chaque groupe (WT, SOD1, SOD1-PL- Low dose et SOD1- PL high dose), collectés dans les cages, ont été analysées à T0 (=T6), T3 semaines (T9) et 10 semaines (T16) soit un total de 55 échantillons.

C1 et C2 ensemble sont désignées « PL » pour la suite de cet essai et des résultats.

Dans une première étape, l'ADN génomique total a été extrait de chaque échantillon fécal par la méthode Godon (Godon et al, Appl Envion. Microbiol, 1997). La qualité de l'ADN extrait a été évaluée après migration des échantillons sur gel d'agarose. La concentration d'ADN a ensuite été déterminée par la technologie Nanodrop.

Les échantillons d'ADNg obtenus ont ensuite été séquencés. L'approche utilisée est le séquençage haut débit du gène codant pour l'ARN ribosomal 16S (zone V3-V4) selon la technologie Illumina (longueur des reads : 150 bases, profondeur de lecture : 1 million de reads).

L'affiliation taxonomique de chaque séquence bactérienne obtenue (ou OTU) a été réalisée à l'aide des bases de données silva_nr99_v138.1 (https://www.arb-silva.de/) et GTDB v202 (https://gtdb.ecogenomic.org/).

Le pipeline ranomaly (Theil S and Rifa E. rANOMALY: AmplicoN wOrkflow for Microbial community AnaLYsis [version 1; peer review: 2 approved]. F1000Research 2021, 10:7 https://doi.org/10.12688/f1000research.27268.1 ), et basé sur le programme dada2, a été utilisé pour traiter l'ensemble des séquences, que ce soit pour l'estimation de l'abondance des phyla/Famille bactériens ou pour réaliser les analyses statistiques (composition, analyses de diversité, analyses différentielles d'abondance).

La comparaison de la diversité et de la richesse bactérienne entre microbiotes issus de souris traitées et non traitées a été réalisée par le calcul de l'indice de Shanon. Les différences de structure des populations (β diversité) ont été mises en évidence par deux méthodes d'analyse multivariée (Anova et Nonmetric Multidimensional Scaling =Method MDS) et une PLS-DA (analyse de régression discriminante des moindres carrés partielles) issue du packages mixOmics.

Les analyses différentielles d'abondances d'OTU ont été réalisé à l'aide des outils de normalisation et d'estimation du package DESeq2 [Love, M.I., Huber, W., Anders, S. Moderated estimation of fold change and dispersion for RNA-seq data with DESeq2 Genome Biology 15(12):550 ; 2014].

### Corrélation Microbiote - Paramètres cliniques

La corrélation potentielle entre chacun des paramètres cliniques et physiologiques mesurés et la composition moléculaire du microbiote a été étudiée pour chacune des souris femelles dont le microbiote a été analysé (5 souris par groupe).

Plusieurs méthodes d'analyse ont été testées, basées sur la corrélation de rang (spearman).

La méthode retenue est l'analyse canonique des corrélations (rcca) proposée par le package mixOmics (Rohart F, Gautier B, Singh A, and Le Cao K-A,2017 : mixOmics: An R package for 'omics feature selection and multiple data integration. PLoS computational biology 13(11):e1005752). Cette méthode permet d'effectuer une validation croisée des résultats d'analyses des corrélations entre l'abondance des OTUs et les différents paramètres cliniques mesurés. Les résultats obtenus sont présentés sous la forme de carte de chaleur (heatmap) montrant les corrélations significatives, positives ou négatives, entre l'abondance des OTU et les paramètres cliniques.

Résultats :
- *Comparaison de la composition du microbiote fécal de souris KO « SOD1 » à celle de souris « Wild Type » (WT)*

L'analyse de l'abondance relative des phyla, ordres, familles voire genres bactériens présents chez les souris SDO1 par rapport aux souris WT montre que la composition du microbiote SOD1 diffère de celle des WT :
- SOD1 ont moins de Firmicutes et plus de Bacteroidetes que les WT
- Parmi les Bacteroidetes, les souris SOD1 hébergent plus de Bacteroidales en particulier Muribaculaceae et plus de Porphyromonaceae dont Muribaculum que les WT
- Parmi les Firmicutes, l'abondance relative des Lachnospiraceae (Shaedlerella et Acetifractor) et Erysipelotrichales est diminuée alors que celle des Lactobacillales comme Limolactobacillus et Oscillospiraceae ainsi que celle des Eggerthelaceae est augmentée par rapport aux souris WT.

L'analyse de la diversité (α diversité) et de la richesse (β diversité) du microbiote fécal de la souris SOD1 par rapport à celui de la souris WT a permis de déterminer qu'il y a :
- Pas de différence significative de la diversité bactérienne (indices : Chao1, Observed, Shannon et Inv Simpson) entre SOD1 et WT ( α diversité).
- Une différence significative (Jaccard : p= 0.01 et Bray Curtis : p=0.01) de la richesse en espèces bactériennes (β diversité). Aucun effet du temps (T0, T6 et T16) n'a été mis en évidence.

L'indice ou la distance de Jaccard et Bray Curtis sont deux métriques bien connues pour mesurer la similarité, la dissimilarité et la diversité entre plusieurs échantillons.

Enfin l'analyse PLS-DA montre que les microbiotes fécaux des souris WT et SOD1 se différencient (Figure 4).

En conclusion, les souris SOD1 présentent un microbiote intestinal perturbé par rapport aux souris sauvages WT , caractérisé par une moindre richesse bactérienne, une plus forte proportion de bactéries à Gram- (Muribaculaceae et Muribaculum) ainsi que de celles des bactéries à Gram + Lactobacillales et Eggerthelaceae mais une plus faible proportion de Lachnospiraceae et Erysipelotrichales.

### - Effet de l'administration de PL à forte ou faible dose chez la souris SOD1

### A - Administration d'une dose forte 10X

L'administration de la composition à dose forte pendant 3 semaines, entraine :
- Une augmentation de l'abondance relative de :
   Bacteroidales en particulier Muribaculaceae
   Oscillospiraceae
   COE1
- Une diminution de l'abondance des :
   Lactobacillaceae (Lactobacillus genus)
   Lachnospiraceae
   L'administration pendant 3 semaines d'une dose forte de la composition semble accentuer les perturbations observées chez la souris SOD1.

### B - Administration d'une dose faible 1X

L'administration d'une dose faible de PL aux souris SOD1 provoque également des modifications de l'abondance relative de certains groupes microbiens. Ainsi, on observe :
- Une augmentation de l'abondance relative de :
   Lachnospiraceae
   Oscillospirales
   GAC-485
- Une diminution de l'abondance relative de :
   Lactobacillale

La dose faible semble ré-équilibrer en partie le microbiote intestinal de la souris SOD1 pour se rapprocher de la composition du microbiote des souris sauvages (WT), notamment en provoquant une augmentation des Lachnospiraceae et une diminution des Lactobacillales. Cet effet semble progressif car il est plus marqué après 10 semaines de traitement qu'après 3 semaines.
- *Comparaison de la diversité et de la richesse bactériennes chez les souris SOD1 traitées et non traitées.*

L'analyse de l'α diversité des microbiotes fécaux n'a pas mis en évidence d'effet significatif de l'administration de PL, à faible et forte dose, sur la diversité bactérienne chez la souris SOD1. Seule une différence significative (indice Shannon, p=0.02) est observée entre les microbiotes fécaux des souris traitées avec la dose faible et celles traitées avec la dose forte de PL. (Figure 5 et 6).

L'analyse de la β diversité a permis de montrer des différences significatives entre souris SOD1 non traitées et souris SOD1 traitées. :
- Contrôles versus PL dose forte (p=0.02 Jaccard et p= 0.01 Bray curtis)
- PL dose faible versus PL dose forte (p = 0.02 Jaccard et p = 0.01 Bray Curtis)
- Pas de différence significative entre contrôle non traité et PL dose faible.
   - *Comparaison entre souris sauvages et souris SOD1 traitées ou non avec différentes doses de*
   PL

L'analyse PLS-DA de la composition de l'ensemble des microbiotes étudiés a permis de montrer que ces microbiotes pouvaient être séparées en 4 groupes distincts, le microbiote des souris traitées avec la faible dose de PL se rapprochant, dans un même cluster du microbiote des souris sauvages (Figure 7).

### - Conclusion

L'étude a permis de montrer que la composition du microbiote des souris SOD1 diffère de celui des souris sauvages, ces altérations touchant la richesse du microbiote plus que sa diversité. Le microbiote des souris SOD1 apparaît perturbé puisque l'abondance relative de certains groupes bactériens majeurs sont altérés. Il se caractérise, en particulier, par une plus forte abondance des Muribaculaceae et les lactobacillales (Oscillospiraceae) au détriment des Lachnospiraceae et Erysipelotrichales. L'analyse PLS-DA a de plus démontré que les microbiotes de souris sauvages et SOD1 étaient séparés.

L'administration d'une dose forte de PL aux souris SOD1 pendant 3 semaines apparait amplifier les différences observées entre souris sauvages et souris SOD1 (augmentation des Muribaculaceae et Oscillospiraceae et diminution des Lachnospiraceae). La composition du microbiote des souris SOD1 traitées avec la forte dose de PL est très nettement différente de celles des contrôles SOD1 mais aussi des souris sauvages, ce groupe de souris traitées à la dose forte s'éloignant largement des autres (microbiote le plus perturbé).

L'administration d'une dose faible de PL aux souris SOD1 pendant 3 à 10 semaines semble ré-équilibrer pour partie les perturbations du microbiote observées chez les souris SOD1 contrôles. On observe en effet une augmentation des Lachnopiraceae et une diminution des Lactobacillales, groupes microbiens affectés chez la souris SOD1 par rapport à la souris sauvage. La composition du microbiote des souris SOD1 traitées avec une dose faible de PL se rapproche ainsi de celle des souris sauvages, les microbiotes de ces deux groupes se trouvant grouper dans un même cluster.

Ces résultats préliminaires sont d'intérêt en ce qui concerne l'administration de la dose la plus faible chez la souris modèle SOD1. A cette dose, les PL semblent effectivement avoir un effet bénéfique sur le microbiote perturbé des souris SOD1.

### Etude de corrélation entre symptômes et composition moléculaire du microbiote chez la souris SOD1 après administration d'une dose faible de Poly-Lysine (PL).

L'objectif de l'étude était d'analyser les corrélations potentielles entre la composition moléculaire du microbiote intestinal et les paramètres cliniques mesurés chez les souris SOD1 versus les souris sauvages ( Wild Type ) et chez les souris SOD1 traitées ou non avec une dose faible de PL pendant 10 semaines.

Cette étude a été réalisée chez les souris femelles dont l'analyse moléculaire du microbiote fécal a été réalisée préalablement (cinq femelles de chaque groupe [WT, SOD1, SOD1-Low dose), collectés dans les cages, à T0 (=T6), T3 semaines (T9) et 10 semaines (T16)].

Les tests ont été effectués au moins 1 h après la première dose quotidienne de composé test ou de véhicule.

Une session d'une journée comprenait un essai d'entraînement de 5 min à 4 RPM sur l'appareil rotatif (AccuScan Instruments, Columbus, USA). Une heure plus tard, les animaux ont été testés pour 3 essais d'accélération consécutifs de 6 min avec la vitesse passant de 0 à 40 RPM sur 360 s et un intervalle inter-essais d'au moins 30 min. La latence pour tomber de la tige a été enregistrée

Etude de corrélation chez la souris SOD1 et Wild Type.

L'analyse de corrélation RCCA (Regularized Canonical Correlation Analysis) a principalement permis de montrer chez les souris WT et SOD1, l'existence d'une corrélation négative importante entre le score clinique, la distance parcourue et le temps de récupération après Rotarod et la famille des Desulfovibrionaceae ainsi qu'une corrélation négative, plus faible, avec la famille des Prevotellaceae. Ainsi plus l'abondance de ces familles bactériennes est élevée plus le score clinique diminue, de même que la distance parcourue et le temps de récupération (Figure 8).

Bien que l'abondance relative de la famille des Desulfovibrionaceae (comme celle des Prevotellaceae) soit faible par rapport à d'autres familles bactériennes, nous avons cependant pu retrouver la présence d'OTU de cette famille chez les souris SOD1 seulement (aucune détection chez les souris Wild Type). Ceci pourrait suggèrer un rôle potentiel des Desulfovibrionaceae dans la physiopathologie chez les souris SOD1.

### Etude de corrélation chez la souris SOD1 traitée ou non traitée avec la dose faible de PL pendant 10 semaines

L'analyse de corrélation RCCA réalisée chez la souris SOD1 contrôle et traitée avec la dose faible de PL a permis de mettre en évidence une corrélation positive forte entre la famille des Lachnospiraceae et la distance parcourue par les souris. Plus cette famille est abondante, plus la distance parcourue par les souris est importante. Sachant qu'un des principaux effets de la PL sur le microbiote intestinal des souris SOD1 est d'augmenter l'abondance des espèces de Lachnospiraceae, la corrélation observée suggère un rôle de cette famille bactérienne dans le rétablissement de la capacité des souris SOD1 à parcourir une distance proche de celle parcourue par les souris Wild Type (Figure 6).

La mise en évidence d'une corrélation négative entre Desulfovibrionaceae et l'importance des symptômes chez la souris SOD1 suggère que les espèces de cette famille, productrices de sulfures, puissent être impliquées dans la physiopathologie, comme cela a été observé dans d'autres pathologies tels que le syndrome du côlon irritable ou les maladies inflammatoires chroniques de l'intestin (Crohn, recto-colite...). Parallèlement, la plus faible abondance d'espèces de la famille des Lachnospiraceae chez la souris SOD1 pourrait se traduire par une diminution de la concentration en butyrate intra-colique, une large part des espèces de Lachnospiraceae étant productrices de ce métabolite aux effets santé démontrés.

L'administration de PL à faible dose pendant 10 semaines s'accompagne de changements au niveau de la composition du microbiote de la souris SOD1, se traduisant, en particulier, par une augmentation de l'abondance des Lachnospiraceae et la disparition des Desulfovibrionaceae. Ceci corrèle avec l'amélioration significative de la capacité des souris à parcourir une distance équivalente à celle des souris Wild Type. L'administration de PL permettrait donc à la fois de ré-équilibrer pour partie le microbiote altéré de la souris SOD1, engendrant des modifications métaboliques et de diminuer la gravité de certains symptômes chez la souris SOD1 voir de rétablir un phénotype normal.

### Etude comparative de l'analyse histopathologique de souris SOD1, SOD1 + faible dose 1X, SOD1+ forte dose 10X.

Des souris SOD1 ont reçu le composé PL décrit à l'exemple 7 à faible (1X) ou à forte dose (10X) ou un véhicule contrôle (n=14/groupe de dosage) à l'âge de 7 à 21 semaines.

De plus, 14 souris de portée de type sauvage (WT) non porteuses ont reçu une dose de véhicule contôle.

Les animaux ont été testés pour leur comportement en champ libre (à l'âge de 6, 9, 13 et 15 semaines), leur performance au Rotarod (à l'âge de 6, 9, 13 et 15 semaines) et leur force de préhension (à l'âge de 6, 9, 13, 15 et 17 semaines). Une analyse ophtalmoscopique a été réalisée à l'âge de 9, 13 et 17 semaines. Le poids corporel, les symptômes cliniques, le délai d'apparition de la maladie et la survie des animaux ont été suivis tout au long de l'étude. Des prélèvements de sang, d'urine et de fèces ont été effectués à l'âge de 6, 9 et 16 semaines. La collecte d'échantillons terminaux a été effectuée lorsqu'un animal répondait à un critère de point final humain ou atteignait l'âge de 150 jours.

L'analyse histopathologique de 30 types de tissus différents a été réalisée.
1. Moelle épinière

| Souris SOD1 | Souris SOD1 + 1X | Souris SOD1 + 10X |
|---|---|---|
| Vacuolisation diffuse minime à modérée chez les mâles, légère à marquée chez les femelles. | Vacuolisation diffuse minimal à marqué chez les hommes, minimal à modéré chez les femmes | Dégénérescence diffuse minime des axones des nerfs rachidiens chez les deux sexes |

2. Cerveau

| Souris SOD1 | Souris SOD1 + 1X | Souris SOD1 + 10X |
|---|---|---|
| Vacuolisation multifocale : légère ou modérée chez les | Vacuolisation légère ou modérée dans les deux sexes | Vacuolisation multifocale minime chez les femelles |

| | | |
|---|---|---|
| mâles, modérée chez les femelles. | | |

3. Nerf sciatique

| Souris SOD1 | Souris SOD1 + 1X | Souris SOD1 + 10X |
|---|---|---|
| Dégénérescence légère ou modérée des axones dans les deux sexes. | Dégénérescence légère ou modérée des axones dans les deux sexes. | Dégénérescence focale minime de l'axone |

4. Muscle Gastrocnémien

| Souris SOD1 | Souris SOD1 + 1X | Souris SOD1 + 10X |
|---|---|---|
| Atrophie minime à modérée chez les deux sexes | Atrophie légère ou modérée chez les mâles, minime à modérée chez les femmes | Atrophie minime chez les mâles et chez les femelles |

5. Tissu adipeux brun, aorte et/ou reins

| Souris SOD1 | Souris SOD1 + 1X | Souris SOD1 + 10X |
|---|---|---|
| Atrophie légère à marquée dans les deux sexes | Atrophie légère à marquée dans les deux sexes | Atrophie diffuse légère chez les hommes et chez les femmes |

6. Rate

| Souris SOD1 | Souris SOD1 + 1X | Souris SOD1 + 10X |
|---|---|---|
| Nécrose des lymphocytes de la pulpe blanche : minime chez les hommes, modérée chez les femmes. | Nécrose minime ou légère des lymphocytes de la pulpe blanche chez les femelles | - |

7. Thymus

| Souris SOD1 | Souris SOD1 + 1X | Souris SOD1 + 10X |
|---|---|---|
| Diminution modérée de la cellularité des lymphocytes chez les mâles. Nécrose lymphocytaire légère ou modérée chez les mâles, modérée chez les femelles. | Diminution modérée de la cellularité des lymphocytes chez les mâles et chez les femelles. Nécrose lymphocytaire minime à marquée chez les femelles. | - |

L'analyse histopathologique montre que l'administration du composé selon l'invention améliore l'état des tissus. Plus particulièrement, la moelle épinière, le cerveau et le muscle gastrocnémien sont les tissus qui présente un état dégradé chez les souris SOD1. On observe clairement que la composition à un effet sur ces tissus en fonction de la dose utilisée.

Ainsi, la composition montre ainsi son effet dose dépendant sur les souris SOD1, montrant ainsi son effet thérapeutique prometteur.

## Revendications

1. Composition comprenant au moins les molécules suivantes :
- acide oléique,
- acide palmitique,
- acide laurique,
- acide linoléique,
- acide azélaïque,
- farnésyl cystéine,
- acide palmitoléïque,
- cholestérol,
- acide thioctique,
- acide myristique,
- acide orotique,
- acide acétique,
- acide butyrique,
- acide lactique,
- acide propionique,
et/ou un sel et/ou un ester et/ou un anhydride d'une ou plusieurs de ces molécules.

2. Composition selon la précédente revendication, **caractérisée en ce qu'**au moins une des molécules de la composition choisie parmi l'acide oléique, l'acide palmitique, l'acide laurique, l'acide linoléique, l'acide azélaïque, l'acide palmitoléïque, l'acide thioctique, l'acide myristique, l'acide orotique, l'acide acétique, l'acide butyrique, l'acide lactique, l'acide propionique, les sels de ces acides, les esters de ces acides et les anhydrides de ces acides, est conjuguée de façon covalente à au moins une molécule d'un polymère choisi parmi la poly-lysine, le poly-éthylène-glycol, la poly-ornithine, la poly-arginine et la poly-histidine.

3. Composition selon l'une des précédente revendications, **caractérisée en ce qu'**elle comprend des micelles dans lesquelles sont encapsulées au moins le farnésyl cystéine et/ou le cholestérol et/ou un ester de ces molécules, préférentiellement au moins une des micelles est formée par des conjugués amphiphiles constitués chacun par au moins une molécule hydrophobe conjuguée de façon covalente à une molécule d'un polymère choisi parmi la poly-lysine, le poly-éthylène-glycol, la Poly-ornithine, la poly-arginine et la poly-histidine.

4. Composition selon la précédente revendication, **caractérisée en ce qu'**au moins une micelle est formée par des conjugués amphiphiles constitués chacun par au moins une molécule choisie parmi l'acide oléique, l'acide palmitique, l'acide laurique, l'acide linoléique, l'acide palmitoléïque, l'acide myristique, les sels, les esters et les anhydrides de ces acides gras, conjuguée de façon covalente à une molécule d'un polymère choisi parmi la poly-lysine, le poly-éthylène-glycol, la Poly-ornithine, la poly-arginine et la poly-histidine.

5. Composition selon l'une des revendications 2 à 4, **caractérisée en ce que** la poly-lysine est une poly-L-lysine de poids moléculaire compris entre 12 000 et 20 000 Da.

6. Composition selon l'une des précédentes revendications, caractérisée en qu'elle comprend au moins :
- A. les conjugués suivants, chaque conjugué étant constitué par une molécule liée de façon covalente à une poly-lysine :
- un ou plusieurs conjugués Oléyl-Poly-L-Lysine
- un ou plusieurs conjugués Palmitic-Poly-L-Lysine
- un ou plusieurs conjugués Lauryl-Poly-L-Lysine
- un ou plusieurs conjugués Azélayl-Poly-L-Lysine
- un ou plusieurs conjugués Palmitoléyl-Poly-L-Lysine
- un ou plusieurs conjugués Thioctyl-Poly-L-Lysine
- un ou plusieurs conjugués Myristyl-Poly-L-Lysine
- un ou plusieurs conjugués Orotyl-Poly-L-Lysine
- un ou plusieurs conjugués Acétate-Poly-L-Lysine
- un ou plusieurs conjugués Butyrate-Poly-L-Lysine
- un ou plusieurs conjugués Lactate-Poly-L-Lysine
- un ou plusieurs conjugués Propionate-Poly-L-Lysine,
- un ou plusieurs conjugués linoléyl-Poly-L-Lysine, et
- B. du farnésylcystéine et du cholestérol, et/ou un ester de ces molécules, encapsulées dans des micelles, préférentiellement lesdites micelles sont formées par un ou plusieurs des conjugués de la liste A.

7. Composition selon la revendication 6, dans laquelle la Poly-L-Lysine est remplacée par une autre poly-lysine ou par le poly-éthylène-glycol, une poly-L-ornithine, une poly-L-arginine ou une poly-L-histidine.

8. Procédé de fabrication d'une composition selon l'une des revendications 1 à 7, **caractérisé en ce qu'**il comprend les étapes suivantes :
- a. créer un premix amphiphile : mélanger dans une solution aqueuse un ou plusieurs conjugués choisis parmi :
- un ou plusieurs conjugués Oléyl-Poly-L-Lysine
- un ou plusieurs conjugués Palmitic-Poly-L-Lysine
- un ou plusieurs conjugués Lauryl-Poly-L-Lysine
- un ou plusieurs conjugués Azéayl-Poly-L-Lysine
- un ou plusieurs conjugués Palmitoléyl-Poly-L-Lysine
- un ou plusieurs conjugués Thioctyl-Poly-L-Lysine
- un ou plusieurs conjugués Myristyl-Poly-L-Lysine
- un ou plusieurs conjugués Orotyl-Poly-L-Lysine
- un ou plusieurs conjugués Acétate-Poly-L-Lysine
- un ou plusieurs conjugués Butyrate-Poly-L-Lysine
- un ou plusieurs conjugués Lactate-Poly-L-Lysine
- un ou plusieurs conjugués Propionate-Poly-L-Lysine,
- un ou plusieurs conjugués linoléyl-Poly-L-Lysine, et
- b. ajouter dans le premix amphiphile au moins du farnésylcystéine et du cholestérol, et mettre sous agitation de façon à former des micelles formées par un ou plusieurs des conjugués du premix amphiphile encapsulant le farnésylcystéine et le cholestérol.

9. Composition selon l'une des revendications 1 à 7, pour son utilisation comme médicament.

10. Composition pour son utilisation selon la revendication 9, où la composition est associée à l'utilisation d'une deuxième composition comprenant les molécules suivantes :
- taurine,
- cystéine,
- méthionine
- spermine
- acide rétinoïque,
- acide pantothénique,
- biotine,
- co-enzyme Q10,
- Glutathion,
- acide ascorbique,
- GABA,
- alpha-tocophérol
et/ou un sel et/ou un ester et/ou un anhydride d'une ou plusieurs de ces molécules.

11. Composition pour son utilisation selon la précédente revendication, **caractérisée en ce que** dans la deuxième composition au moins une des molécules de la composition choisie parmi la taurine, la spermine, l'acide pantothénique, l'acide rétinoïque, la biotine, le Glutathion, l'acide ascorbique, le GABA, et l'alpha-tocophérol, les sels de ces molécules, les esters de ces molécules, et les anhydrides de ces molécules, est conjuguée de façon covalente à une molécule d'un polymère choisi parmi la poly-lysine, le poly-éthylène-glycol, la poly-ornithine, la poly-arginine et la poly-histidine.

12. Composition pour son utilisation selon l'une des revendications 10 ou 11, **caractérisée en ce que** dans la deuxième composition la cystéine et la méthionine sont chacune conjuguée à une molécule d'un polymère choisi parmi la poly-lysine, le poly-éthylène-glycol, la Poly-ornithine, la poly-arginine et la poly-histidine, par polymérisation, ladite molécule et ledit polymère formant un copolymère.

13. Composition pour son utilisation selon l'une des revendications 10 à 12, **caractérisée en ce que** dans la deuxième composition le co-enzyme Q10 est encapsulé dans des micelles.

14. Composition pour son utilisation selon précédente revendication, **caractérisée en ce qu'**au moins une micelle de la deuxième composition est formée par des conjugués amphiphiles constitués chacun par au moins une molécule conjuguée de façon covalente à une molécule d'un polymère choisi parmi la poly-lysine, le poly-éthylène-glycol, la poly-ornithine, la poly-arginine et la poly-histidine, préférentiellement lesdits conjugués amphiphiles sont constitués chacun par au moins une molécule choisie parmi la taurine, la spermine, l'acide pantothénique, la biotine, le Glutathion, l'acide ascorbique, le GABA, et l'alpha-tocophérol, les sels de ces molécules, les esters de ces molécules et les anhydrides de ces molécules.

15. Composition pour son utilisation selon l'une des revendications 10 à 14, dans la prévention et/ou le traitement d'une dysbiose pathologique du microbiote intestinal ou d'une maladie de l'intestin associée à une dysbiose pathologique du microbiote intestinal, préférentiellement ladite dysbiose est choisie parmi la maladie de Crohn, les maladies inflammatoires chronique de l'intestin, la rectocolite hémorragique, le syndrome de l'intestin irritable, la colite ulcéreuse, la polyarthrite rhumatoïde et l'intolérance au gluten.

## Patentansprüche

1. Zusammensetzung, umfassend mindestens die folgenden Moleküle:
- Ölsäure,
- Palmitinsäure,
- Laurinsäure,
- Linolsäure,
- Azelainsäure,
- Farnesylcystein,
- Palmitoleinsäure,
- Cholesterin,
- Thioctsäure,
- Myristinsäure,
- Orotsäure,
- Essigsäure,
- Buttersäure,
- Milchsäure,
- Propionsäure,
und/oder ein Salz und/oder ein Ester und/oder ein Anhydrid eines oder mehrerer dieser Moleküle.

2. Zusammensetzung nach dem vorstehenden Anspruch,
**dadurch gekennzeichnet, dass** mindestens eines der Moleküle der Zusammensetzung, ausgewählt aus Ölsäure, Palmitinsäure, Laurinsäure, Linolsäure, Azelainsäure, Palmitoleinsäure, Thioctsäure, Myristinsäure, Orotsäure, Essigsäure, Buttersäure, Milchsäure, Propionsäure, den Salzen dieser Säuren, den Estern dieser Säuren und den Anhydriden dieser Säuren, an mindestens ein Molekül eines Polymers, ausgewählt aus Polylysin, Polyethylenglykol, Polyornithin, Polyarginin und Polyhistidin, kovalent konjugiert ist.

3. Zusammensetzung nach einem der vorstehenden Ansprüche,
**dadurch gekennzeichnet, dass** sie Mizellen umfasst, in denen mindestens Farnesylcystein und/oder Cholesterin und/oder ein Ester dieser Moleküle eingekapselt ist, vorzugsweise mindestens eine der Mizellen durch amphiphile Konjugate ausgebildet ist, die jeweils durch mindestens ein hydrophobes Molekül gebildet sind, das an ein Molekül eines Polymers, ausgewählt aus Polylysin, Polyethylenglykol, Polyornithin, Polyarginin und Polyhistidin, kovalent konjugiert ist.

4. Zusammensetzung nach dem vorstehenden Anspruch,
**dadurch gekennzeichnet, dass** mindestens eine Mizelle durch amphiphile Konjugate ausgebildet ist, die jeweils durch mindestens ein Molekül gebildet sind, ausgewählt aus Ölsäure, Palmitinsäure, Laurinsäure, Linolsäure, Palmitoleinsäure, Myristinsäure, den Salzen, den Estern und den Anhydriden dieser Fettsäuren, und an ein Molekül eines Polymers kovalent konjugiert ist, ausgewählt aus Polylysin, Polyethylenglykol, Polyornithin, Polyarginin und Polyhistidin.

5. Zusammensetzung nach einem der Ansprüche 2 bis 4,
**dadurch gekennzeichnet, dass** Polylysin ein Poly-L-Lysin mit einem Molekulargewicht zwischen 12.000 und 20.000 Da ist.

6. Zusammensetzung nach einem der vorstehenden Ansprüche,
**dadurch gekennzeichnet, dass** sie mindestens umfasst:
- A. die folgenden Konjugate, wobei jedes Konjugat durch ein Molekül gebildet ist, das an ein Polylysin kovalent gebunden ist:
- ein oder mehrere Oleylpoly-L-Lysinkonjugate
- ein oder mehrere Palmitinpoly-L-Lysinkonjugate
- ein oder mehrere Laurylpoly-L-Lysinkonjugate
- ein oder mehrere Azelaylpoly-L-Lysinkonjugate
- ein oder mehrere Palmitoleylpoly-L-Lysinkonjugate
- ein oder mehrere Thioctylpoly-L-Lysinkonjugate
- ein oder mehrere Myristylpoly-L-Lysinkonjugate
- ein oder mehrere Orotylpoly-L-Lysinkonjugate
- ein oder mehrere Acetatpoly-L-Lysinkonjugate
- ein oder mehrere Butyratpoly-L-Lysinkonjugate
- ein oder mehrere Lactatpoly-L-Lysinkonjugate
- ein oder mehrere Propionatpoly-L-Lysinkonjugate,
- ein oder mehrere Linoleylpoly-L-Lysinkonjugate und
- B. Farnesylcystein und Cholesterin und/oder ein Ester dieser Moleküle, eingekapselt in Mizellen, vorzugsweise wobei die Mizellen durch ein oder mehrere der Konjugate aus Liste A ausgebildet sind.

7. Zusammensetzung nach Anspruch 6, wobei das Poly-L-Lysin durch ein anderes Poly-Lysin oder durch Polyethylenglykol, Poly-L-Ornithin, Poly-L-Arginin oder Poly-L-Histidin ersetzt ist.

8. Verfahren zum Herstellen einer Zusammensetzung nach einem der Ansprüche 1 bis 7, **dadurch gekennzeichnet, dass** es die folgenden Schritte umfasst:
- a. Erstellen einer amphiphilen Vormischung: Mischen, in einer wässrigen Lösung, eines oder mehrerer Konjugate, ausgewählt aus:
- einem oder mehreren Oleylpoly-L-Lysinkonjugaten
- einem oder mehreren Palmitinpoly-L-Lysinkonjugaten
- einem oder mehreren Laurylpoly-L-Lysinkonjugaten
- einem oder mehreren Azeaylpoly-L-Lysinkonjugaten
- einem oder mehreren Palmitoleylpoly-L-Lysinkonjugaten
- einem oder mehreren Thioctylpoly-L-Lysinkonjugaten
- einem oder mehreren Myristylpoly-L-Lysinkonjugaten
- einem oder mehreren Orotylpoly-L-Lysinkonjugaten
- einem oder mehreren Acetatpoly-L-Lysinkonjugaten
- einem oder mehreren Butyratpoly-L-Lysinkonjugaten
- einem oder mehreren Lactatpoly-L-Lysinkonjugaten
- einem oder mehreren Propionatpoly-L-Lysinkonjugaten,
- einem oder mehreren Linoleylpoly-L-Lysinkonjugaten und
- b. Zugeben von mindestens Farnesylcystein und Cholesterin zu der amphiphilen Vormischung und Rühren, um Mizellen auszubilden, die durch ein oder mehrere der Konjugate der amphiphilen Vormischung ausgebildet sind, die Farnesylcystein und Cholesterin einkapseln.

9. Zusammensetzung nach einem der Ansprüche 1 bis 7, für die Verwendung als Arzneimittel.

10. Zusammensetzung für die Verwendung nach Anspruch 9, wobei die Zusammensetzung mit der Verwendung einer zweiten Zusammensetzung kombiniert wird, umfassend die folgenden Moleküle:
- Taurin,
- Cystein,
- Methionin
- Spermin
- Retinsäure,
- Pantothensäure,
- Biotin,
- Coenzym Q10,
- Glutathion,
- Ascorbinsäure,
- GABA,
- Alpha-Tocopherol
und/oder ein Salz und/oder ein Ester und/oder ein Anhydrid eines oder mehrerer dieser Moleküle.

11. Zusammensetzung für die Verwendung nach dem vorstehenden Anspruch, **dadurch gekennzeichnet, dass** bei der zweiten Zusammensetzung mindestens eines der Moleküle der Zusammensetzung, ausgewählt aus Taurin, Spermin, Pantothensäure, Retinsäure, Biotin, Glutathion, Ascorbinsäure, GABA und Alpha-Tocopherol, den Salzen dieser Moleküle, den Estern dieser Moleküle und den Anhydriden dieser Moleküle, an ein Molekül eines Polymers, ausgewählt aus Polylysin, Polyethylenglykol, Polyornithin, Polyarginin und Polyhistidin, kovalent konjugiert ist.

12. Zusammensetzung für die Verwendung nach einem der Ansprüche 10 oder 11, **dadurch gekennzeichnet, dass** bei der zweiten Zusammensetzung Cystein und Methionin jeweils durch Polymerisation an ein Molekül eines Polymers, ausgewählt aus Polylysin, Polyethylenglykol, Polyornithin, Polyarginin und Polyhistidin, konjugiert sind, wobei das Molekül und das Polymer ein Copolymer ausbilden.

13. Zusammensetzung für die Verwendung nach einem der Ansprüche 10 bis 12, **dadurch gekennzeichnet, dass** bei der zweiten Zusammensetzung das Coenzym Q10 in Mizellen eingekapselt ist.

14. Zusammensetzung für die Verwendung nach dem vorstehenden Anspruch, **dadurch gekennzeichnet, dass** mindestens eine Mizelle der zweiten Zusammensetzung durch amphiphile Konjugate ausgebildet ist, die jeweils durch mindestens ein Molekül gebildet sind, das an ein Molekül eines Polymers, ausgewählt aus Polylysin, Polyethylenglykol, Polyornithin, Polyarginin und Polyhistidin, kovalent konjugiert ist, wobei die amphiphilen Konjugate vorzugsweise jeweils durch mindestens ein Molekül gebildet sind, ausgewählt aus Taurin, Spermin, Pantothensäure, Biotin, Glutathion, Ascorbinsäure, GABA und Alpha-Tocopherol, den Salzen dieser Moleküle, den Estern dieser Moleküle und den Anhydriden dieser Moleküle.

15. Zusammensetzung für die Verwendung nach einem der Ansprüche 10 bis 14 bei der Vorbeugung und/oder der Behandlung einer pathologischen Dysbiose der Darmmikrobiota oder einer Darmerkrankung, die mit einer pathologischen Dysbiose der Darmmikrobiota verbunden ist, vorzugsweise wobei die Dysbiose ausgewählt ist aus Morbus Crohn, chronisch entzündlichen Darmerkrankungen, ulzerativer Kolitis, Reizdarmsyndrom, Colitis ulcerosa, rheumatoider Arthritis und Zöliakie.

## Claims

1. A composition comprising at least the following molecules:
- oleic acid,
- palmitic acid,
- lauric acid,
- linoleic acid,
- azelaic acid,
- farnesyl cysteine,
- palmitoleic acid,
- cholesterol,
- thioctic acid,
- myristic acid,
- orotic acid,
- acetic acid,
- butyric acid,
- lactic acid,
- propionic acid,
and/or a salt and/or an ester and/or an anhydride of one or more of these molecules.

2. The composition according to the preceding claim, **characterized in that** at least one of the molecules of the composition selected from among oleic acid, palmitic acid, lauric acid, linoleic acid, azelaic acid, palmitoleic acid, thioctic acid, myristic acid, orotic acid, acetic acid, butyric acid, lactic acid, propionic acid, salts of these acids, esters of these acids and anhydrides of these acids, is covalently conjugated to at least one molecule of a polymer selected from among polylysine, polyethylene glycol, polyornithine, polyarginine and polyhistidine.

3. The composition according to either of the preceding claims,
**characterized in that** it comprises micelles in which are encapsulated at least farnesyl cysteine and/or cholesterol and/or an ester of these molecules, preferentially at least one of the micelles is formed by amphiphilic conjugates each consisting of at least one hydrophobic molecule covalently conjugated to a molecule of a polymer selected from among polylysine, polyethylene glycol, polyornithine, polyarginine and polyhistidine.

4. The composition according to the preceding claim, **characterized in that** at least one micelle is formed by amphiphilic conjugates each consisting of at least one molecule selected from among oleic acid, palmitic acid, lauric acid, linoleic acid, palmitoleic acid, myristic acid, salts, esters and anhydrides of these fatty acids, covalently conjugated to a molecule of a polymer selected from among polylysine, polyethylene glycol, polyornithine, polyarginine and polyhistidine.

5. The composition according to any of claims 2 to 4, **characterized in that** the polylysine is a poly-L-lysine with a molecular weight of between 12000 and 20000 Da.

6. The composition according to any of the preceding claims, **characterized in that** it comprises at least:
- A. the following conjugates, each conjugate consisting of a molecule covalently bonded to a polylysine:
- one or more oleyl-poly-L-lysine conjugates
- one or more palmitic-poly-L-lysine conjugates
- one or more lauryl-poly-L-lysine conjugates
- one or more azelayl-poly-L-lysine conjugates
- one or more palmitoleyl-poly-L-lysine conjugates
- one or more thioctyl-poly-L-lysine conjugates
- one or more myristyl-poly-L-lysine conjugates
- one or more orotyl-poly-L-lysine conjugates
- one or more acetate-poly-L-lysine conjugates
- one or more buyrate-poly-L-lysine conjugates
- one or more lactate-poly-L-lysine conjugates
- one or more propionate-poly-L-lysine conjugates,
- one or more linoleyl-poly-L-lysine conjugates, and
- B. farnesyl cysteine and cholesterol, and/or an ester of these molecules, encapsulated in micelles, preferentially the micelles being formed by one or more of the conjugates in list A.

7. The composition according to claim 6, wherein the poly-L-lysine is replaced with another polylysine or with polyethylene glycol, a poly-L-ornithine, a poly-L-arginine or a poly-L-histidine.

8. A method for producing a composition according to any of claims 1 to 7, **characterized in that** it comprises the following steps:
- a. creating an amphiphilic premix: mixing, in an aqueous solution, one or more conjugates selected from among:
- one or more oleyl-poly-L-lysine conjugates
- one or more palmitic-poly-L-lysine conjugates
- one or more lauryl-poly-L-lysine conjugates
- one or more azelayl-poly-L-lysine conjugates
- one or more palmitoleyl-poly-L-lysine conjugates
- one or more thioctyl-poly-L-lysine conjugates
- one or more myristyl-poly-L-lysine conjugates
- one or more orotyl-poly-L-lysine conjugates
- one or more acetate-poly-L-lysine conjugates
- one or more buyrate-poly-L-lysine conjugates
- one or more lactate-poly-L-lysine conjugates
- one or more propionate-poly-L-lysine conjugates,
- one or more linoleyl-poly-L-lysine conjugates, and
- b. adding at least farnesyl cysteine and cholesterol to the amphiphilic premix and stirring so as to form micelles formed by one or more of the conjugates of the amphiphilic premix encapsulating the farnesyl cysteine and cholesterol.

9. The composition according to any of claims 1 to 7, for use thereof as a drug.

10. The composition for use thereof according to claim 9, wherein the composition is associated with the use of a second composition comprising the following molecules:
- taurine,
- cysteine,
- methionine
- spermine
- retinoic acid,
- pantothenic acid,
- biotin,
- coenzyme Q10,
- glutathione,
- ascorbic acid,
- GABA,
- alpha-tocopherol
and/or a salt and/or an ester and/or an anhydride of one or more of these molecules.

11. The composition for use thereof according to the preceding claim, **characterized in that,** in the second composition, at least one of the molecules of the composition selected from among taurine, spermine, pantothenic acid, retinoic acid, biotin, glutathione, ascorbic acid, GABA, and alpha-tocopherol, salts of these molecules, esters of these molecules, and anhydrides of these molecules is covalently conjugated to a molecule of a polymer selected from among polylysine, polyethylene glycol, polyornithine, polyarginine and polyhistidine.

12. The composition for use thereof according to any of claims 10 or 11, **characterized in that,** in the second composition, cysteine and methionine are each conjugated to a molecule of a polymer selected from among polylysine, polyethylene glycol, polyornithine, polyarginine and polyhistidine by polymerization, the molecule and the polymer forming a copolymer.

13. The composition for use thereof according to any of claims 10 to 12, **characterized in that,** in the second composition, coenzyme Q10 is encapsulated in micelles.

14. The composition for use thereof according to the preceding claim, **characterized in that** at least one micelle of the second composition is formed by amphiphilic conjugates each consisting of at least one molecule covalently conjugated to a molecule of a polymer selected from among polylysine, polyethylene glycol, polyornithine, polyarginine and polyhistidine, preferentially the amphiphilic conjugates each consisting of at least one molecule selected from among taurine, spermine, pantothenic acid, biotin, glutathione, ascorbic acid, GABA and alpha-tocopherol, the salts of these molecules, the esters of these molecules and the anhydrides of these molecules.

15. The composition for use thereof according to any of claims 10 to 14, in the prevention and/or treatment of pathological dysbiosis of the gut microbiota or an intestinal disease associated with pathological dysbiosis of the gut microbiota, wherein preferentially the dysbiosis is selected from among Crohn's disease, chronic inflammatory bowel diseases, hemorrhagic rectocolitis, irritable bowel syndrome, ulcerative colitis, rheumatoid arthritis and gluten intolerance.
